# EUROPEAN PATENT SPECIFICATION

(11) **EP 1 643 989 B1**
(45) Date of publication and mention of the grant of the patent: **28.04.2010**
(21) Application number: 04736392.4
(22) Date of filing: 09.06.2004
(51) Int. Cl.: A61K 31/4164, C07D 233/22, C07D 401/06, C07D 403/06, A61P 35/00

(54) **CIS-IMIDAZOLINES AS MDM2 INHIBITORS**
CIS-IMIDAZOLINE ALS MDM2-HEMMER
CIS-IMIDAZOLINES UTILISEES EN TANT QU'INHIBITEURS DE MDM2

(30) Priority: 17.06.2003 US 479563 P
(43) Date of publication of application: 12.04.2006
(73) Proprietor: F. Hoffmann-La Roche AG, 4070 Basel (CH)
(72) Inventor: FOTOUHI, Nader, Basking Ridge, NJ 07920 (US); LIU, Emily, Aijun, Nutley, NJ 07110 (US); VU, Binh, Thanh, North Caldwell, NJ 07006 (US)
(74) Representative: Halbig, Dirk
(86) International application number: PCT/EP2004/006211
(87) International publication number: WO 2005/002575

(56) References cited:
- EP-A- 0 433 682
- WO-A-03/051359
- WO-A-03/051360

## Description

This invention is related to at least one compound selected from a compound of formula I 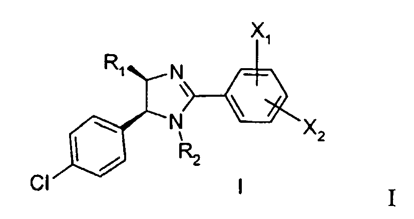 and the pharmaceutically acceptable salts and esters thereof, wherein X₁, X₂, R₁ and R₂ are described within this application. This compound is believed to inhibit the interaction of MDM2 protein with a p53-like peptide and have antiproliferative activity

p53 is a tumor suppresser protein that plays a central role in protection against development of cancer. It guards cellular integrity and prevents the propagation of permanently damaged clones of cells by the induction of growth arrest or apoptosis. At the molecular level, p53 is a transcription factor that can activate a panel of genes implicated in the regulation of cell cycle and apoptosis. p53 is a potent cell cycle inhibitor which is tightly regulated by MDM2 at the cellular level. MDM2 and p53 form a feedback control loop. MDM2 scan bind p53 and inhibit its ability to transactivate p53-regulated genes. In addition, MDM2 mediates the ubiquitin-dependent degradation of p53. p53 can activate the expression of the MDM2 gene, thus raising the cellular level of MDM2 protein. This feedback control loop insures that both MDM2 and p53 are kept at a low level in normal proliferating cells. MDM2 is also a cofactor for E2F, which plays a central role in cell cycle regulation.

The ratio of MDM2 to p53 (E2F) is dysregulated in many cancers. Frequently occurring molecular defects in the p16INK4/p19ARF locus, for instance, have been shown to affect MDM2 protein degradation. Inhibition of MDM2-p53 interaction in tumor cells with wild-type p53 should lead to accumulation of p53, cell cycle arrest and/or apoptosis. MDM2 antagonists, therefore, can offer a novel approach to cancer therapy as single agents or in combination with a broad spectrum of other antitumor therapies. The feasibility of this strategy has been shown by the use of different macromolecular tools for inhibition of MDM2-p53 interaction (e.g. antibodies, antisense oligonucleotides, peptides). MDM2 also binds E2F through a conserved binding region as p53 and activates E2F-dependent transcription of cyclin A, suggesting that MDM2 antagonists might have effects in p53 mutant cells.

Wells et al. J. Org. Chem., 1972, 37, 2158-2161, report synthesis of imidazolines. Hunter et al. Can. J. Chem., 1972, Vol. 50, 669-77, report the preparation of amarine and isoamarine compounds which had previously been studied for chemiluminescence (McCapra et al. Photochem. and Photobiol. 1965, 4, 1111-1121). Zupanc et al. Bull. Soc. Chem. & Tech. (Yugoslavia) 1980-81, 27/28, 71-80, report the use of triaryl imidazolines as starting materials in the preparation of EDTA derivatives. EP 363 061 to Matsumoto reports imidazoline derivatives useful as immunomodulators. The compounds were indicated to have low toxicity. Treatment and/or prevention of rheumatoid arthritis, multiple sclerosis, systemic lupus, erythemathodes, and rheumatic fever were implicated. WO 00/78725 to Choueiry et al. report a method for making substituted amidine compounds, and indicate that imidazoline-type compounds may be useful in the treatment of diabetes or related diseases involving impaired glucose disposal.

The present invention is directed to at least one compound selected from a compound a compound of formula I 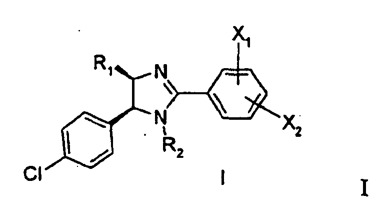 wherein
- X₁ and: X₂ are each independently selected from the group consisting of hydrogen, -OR₃, -SR₄ -NR₅R₆ -CONR₇R₈, -COOR₉, halogen, nitro, trifluoromethyl, C₁-C₆alkyl, C₁-C₆alkyl substituted by R₁₀ and C₃-C₈cycloalkyl;
- R₁: is selected from the group consisting of C1-C8 alkyl; C1-C4 alkyl attached to C4-C8 cycloalkyl; and C4-C8 cycloalkyl;
- R₂: is H or -C=OR₉;
- R₃: is selected from the group consisting of hydrogen, C₁-C₆ alkyl, C₁-C₆ alkyl substituted by R₈, and C₃-C₈ cycloalkyl;
- R₄ is: selected from the group consisting of hydrogen and C₁-C₆ alkyl;
- R₅ and R₆: are each independently selected from the group consisting of hydrogen, C₁-C₆ alkyl and C₃-C₈ cycloalkyl; or
- R₅ and R₆: together with the two carbon atoms and the bonds between them from the benzene ring to which they are substituted form a ring selected from a 5- or 6- membered unsaturated ring or a 5- or 6-membered saturated ring that contains at least one hetero atom selected from S, N and O;
- R₇: is selected from the group consisting of hydrogen, C₁-C₆ alkyl and C₃-C₈ cycloalkyl;
- R₈: is selected from the group consisting of -CONR₅R₆, -NR₅R₆, COOR₇, aryl, halogen, C₁-C₆ alkoxy, morpholinyl and heterocycles;
- R₉: is selected from C1-C4 alkyl, -CH=CHCOOH, -NHCH₂CH₂R₁₀ -N(CH₂CH₂OH)CH₂CH₂OH, -N(CH₃)CH₂CH₂NCH₃, -N(CH₃)CH₂CH₂N(CH₃)CH₃, saturated 4-, 5- and 6-membered rings, and saturated and unsaturated 5- and 6- membered rings containing at least one hetero atom wherein the hetero atom is selected from S, N and O and being optionally substituted with a group selected from C₁-C₆ alkyl, -C=O-R₁₁, -OH, C₁-C₆ alkyl substituted with hydroxy, C₁-C₆ alkyl substituted with -NH₂, N-C₁-C₆ alkyl, -SO₂C-H₃, =O, -CH₂C=OCH₃, and 5- and 6-membered saturated rings containing at least one hetero atom selected from S, N and O;
- R₁₀: is selected from -N(CH₃)CH₃, -NHCH₂CH₂NH₂, -NH₂, morpholinyl and piperazinyl; and
- R₁₁: is selected from H, C₁-C₆ alkyl, -NH₂, -N-C₁-C₆ alkyl, C₁-C₆ alkyl substituted with hydroxy, and C₁-C₆ alkyl substituted with NH₂,
whereby each cycloalkyl represents a non-aromatic, partially or completely saturated cyclic hydrocarbon group
or a pharmaceutically acceptable salt or ester thereof.

The present invention is directed to at least one compound selected from a compound of formula I 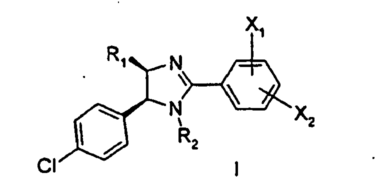 and the pharmaceutically acceptable salts and esters thereof, wherein
- X₁ and X₂: are each independently selected from the group consisting of hydrogen, -OR₃, -NR_{S}R₆, -CONR₇R₈, -COOR₉, halogen, nitro, trifluoromethyl, C₁-C₆ alkyl, C₁-C₆ alkyl substituted by R₁₀, C₃-C₈ cycloalkyl;
- R₁ is: selected from the group consisting of C1-C8 alkyl, C1-C4 alkyl attached to C4-C8 cycloalkyl, and C4-C8 cycloalkyl;
- R₂: is H or -C=OR₉;
- R₃: is selected from the group consisting of hydrogen, C₁-C₆ alkyl, C₁-C₆ alkyl substituted by R₈, and C₃-C₈ cycloalkyl;
- R₄: is selected from the group consisting of hydrogen and C₁-C₆ alkyl;
- R₅ and R₆: are each independently selected from the group consisting of hydrogen, C₁-C₆ alkyl and C₃-C₈ cycloalkyl; or
- R₅ and R₆: together with the two carbon atoms and the bonds between them from the benzene ring to which they are substituted form a ring selected from a 5- or 6- membered unsaturated ring or a 5- or 6-membered saturate ring that contains at least one hetero atom selected from S, N and O;
- R₇: is selected from the group consisting of hydrogen, C₁-C₆ alkyl and C₃-C₈ cycloalkyl;
- R₈: is selected from the group consisting of -CONR₅R₆, -NR₅R₆, COOR₇, aryl, halogen, C₁-C₆ alkoxy, morpholinyl and heterocycles;
- R₉: is selected from C1-C4 alkyl, -CH=CHCOOH, -NHCH₂CH₂R₁₀, -N(CH₂CH₂OH)CH₂CH₂OH, -N(CH₃)CH₂CH₂NCH₃, -N(CH₃)CH₂CH₂N(CH₃)CH₃, saturated 4-, 5- and 6-membered rings, and saturated and unsaturated 5- and 6- membered rings containing at least one hetero atom wherein the hetero atom is selected from S, N and O and being optionally substituted with a group selected from C₁-C₆ alkyl, -C=O-R₁₁, -OH, C₁-C₆ alkyl substituted with hydroxy, C₁-C₆ alkyl substituted with -NH₂, N-C₁-C₆ alkyl, -SO₂CH₃, =O, -CH₂C=OCH₃, and 5- and 6-membered saturated rings containing at least one hetero atom selected from S, N and O;
- R₁₀: is selected from -N(CH₃)CH₃, -NHCH₂CH₂NH₂, -NH₂, morpholinyl and piperazinyl;
and
- R₁₁: is selected from H, C₁-C₆ alkyl, -NH₂, -N-C₁-C₆ alkyl, C₁-C₆ alkyl substituted with hydroxy, and C₁-C₆ alkyl substituted with NH_{2.}
or a pharmaceutically acceptable salt or ester thereof.

When R₂ = H, the compounds of the formula I may exist as a mixture of 2 stereoisomers IA and IB. Therefore, this invention includes these 2 isomers (when R₂ = H).

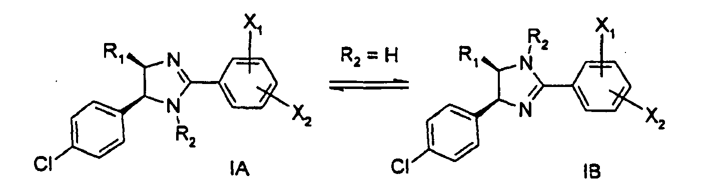

Preferably, R1 is C₁-C₈ alkyl, C₁-C₄ alkyl attached to C₄-C₈ cycloalkyl. X₁ is selected from the group consisting of ethoxy, isopropoxy, 2-fluoroethoxy and -OCH₂CF₃ at the ortho position. X₂ is selected from the group consisting of methoxy, ethoxy and trifluoromethyl at the para position. The compound of claim 1 wherein R₉ is selected from morpholinyl, piperazinyl, piperadinyl, cyclopentyl, cyclohexyl, thiophenyl, isoxazlyl and furanyl, piperazinyl substituted with at least one group selected from C1-C3 alkyl, -C1-C2 alkoxy, -C=OCH₃, -SO₂CH₃, -C=O, -OH, -CH₂NH₂, -C=OCH₂NH₂, -C=OCH₂OH, -C=OC(OH)CH₂OH, -CH₂C(OH)-CH₂OH, -C=ON(CH₂-)_{2,} -C=ONH2, -C=ON(CH₃)CH₃, -C=OCH(CH₃)₂, -CH₂C=CH₃, -CH₂CH(OH)CH₃, -CH(CH₃)CH(OH)CH₃ and piperidinyl substituted with at least one group selected from C1-C3 alkyl, -C1-C2 alkoxy, -C=OCH₃, -SO₂CH₃, -C=O, -OH, -CH₂NH₂, -C=CH₂NH₂, -C=OCH₂OH, -C=OC(OH)CH₂OH, -CH₂C(OH) CH₂OH, -C=ON(CH₂)₂, -C=ONH₂, and -C=ON(CH₃)CH₃, -N(CH₃)CH₃, pyrrolidinyl and piperadinyl.

"Effective amount" means an amount that is effective to prevent, alleviate or ameliorate symptoms of disease or prolong the survival of the subject being treated.

"Halogen" means fluorine, chlorine, bromine or iodine.

"Hetero atom" means an atom selected from N, O and S.

"IC₅₀" refers to the concentration of a particular compound required to inhibit 50% of a specific measured activity. IC₅₀ can be measured, inter alia, as is described subsequently.

"Alkyl" denotes a straight-chained or branched saturated aliphatic hydrocarbon. "Lower alkyl" groups denote C1-C6 alkyl groups and include methyl, ethyl, propyl, isopropyl, butyl, t-butyl, 2-butyl, pentyl, hexyl, and the like. Generally, lower alkyl is preferably C1-C4 alkyl, and more preferably C1-C3 alkyl.

"Cycloalkyl" means a non-aromatic, partially or completely saturated cyclic aliphatic hydrocarbon group containing 3 to 8 atoms.

"Heterorycle" means a 3 to 10 member saturated or partially saturated non-aromatic monovalent cyclic radical having from 1 to 3 heteroatoms selected from nitrogen, oxygen or sulfur, or a combination thereof.

"Alkoxy" denotes -O-alkyl. "Lower alkoxy" denotes -O-lowed alkyl.

"Saturated 4-, 5- and 6-membered rings" refers to cycloalkyl structures having 4, 5 and 6 carbons on the ring structure, respectively. Specifically, they refer to cyclobutyl, cyclopentyl and cyclohexyl respectively.

"Saturated or unsaturated 5- and 6-membered rings containing at least one hetero atom wherein the hetero atom is selected from S, N and O" refers to a cyclic structure having 5 and 6 carbons on the ring respectively, such that the ring may be saturated or unsaturated, and wherein seach such structure contains one or two hetero atoms. Examples of saturated or unsatureated 5- and 6-membered rings containing at least one hetero atom wherein the hetero atom is selected from S, N and O are pyrrolidine, piperidine, piperazine, morpholine, pyrrole, and imidazole.

"Pharmaceutically acceptable ester" refers to a conventionally esterified compound of formula I having a carboxyl group, which esters retain the biological effectiveness and properties of the compounds of formula I and are cleaved in vivo (in the organism) to the corresponding active carboxylic acid.

Information concerning esters and the use of esters for the delivery of pharmaceutical compounds is available in Design of Prodrugs. Bundgaard H ed. (Elsevier, 1985). *See also,* H. Ansel et. al., Pharmaceutical Dosage Forms and Drug Delivery Systems (6th Ed. 1995) at pp. 108-109; Krogsgaard-Larsen, et. al., Textbook of Drug Design and Development (2d Ed. 1996) at pp. 152-191.

"Pharmaceutically acceptable salt" refers to conventional acid-addition salts or base-addition salts that retain the biological effectiveness and properties of the compounds of the present invention and are formed from suitable non-toxic organic or inorganic acids or organic or inorganic bases. Sample acid-addition salts include those derived from inorganic acids such as hydrochloric acid, hydrobromic acid, hydroiodic acid, sulfuric acid, sulfamic acid, phosphoric acid and nitric acid, and those derived from organic acids such as p-toluenesulfonic acid, salicylic acid, methanesulfonic acid, oxalic acid, succinic acid, citric acid, malic acid, lactic acid, fumaric acid, and the like. Sample base-addition salts include those derived from ammonium, potassium, sodium and, quaternary ammonium hydroxides, such as for example, tetramethylammonium hydroxide. Chemical modification of a pharmaceutical compound (i.e. drug) into a salt is a technique well known to pharmaceutical chemists to obtain improved physical and chemical stability, hygroscopicity, flowability and solubility of compounds. *See, e.g.,* H. Ansel et. al., Pharmaceutical Dosage Forms and Drug Delivery Systems (6th Ed. 1995) at pp. 196 and 1456-1457.

"Pharmaceutically acceptable," such as pharmaceutically acceptable carrier, excipient, etc., means pharmacologically acceptable and substantially non-toxic to the subject to which the particular compound is administered.

"Substituted," as in substituted alkyl, means that the substitution can occur at one or more positions and, unless otherwise indicated, that the substituents at each substitution site are independently selected from the specified options.

A "therapeutically effective amount" of a compound in accordance with this invention means an amount of compound that is effective to prevent, alleviate or ameliorate symptoms of disease or prolong the survival of the subject being treated. Determination of a therapeutically effective amount is within the skill in the art.

The therapeutically effective amount or dosage of a compound according to this invention can vary within wide limits and may be determined in a manner known in the art. Such dosage will be adjusted to the individual requirements in each particular case including the specific compound(s) being administered, the route of administration, the condition being treated, as well as the patient being treated. In general, in the case of oral or parenteral administration to adult humans weighing approximately 70 Kg, a daily dosage of about 10 mg to about 10,000 mg, preferably from about 200 mg to about 1,000 mg, should be appropriate, although the upper limit may be exceeded when indicated. The daily dosage can be administered as a single dose or in divided doses, or for parenteral administration, it may be given as continuous infusion.

The compounds of the present invention are useful in the treatment or control of cell proliferative disorders, in particular oncological disorders. These compounds and formulations containing said compounds may be useful in the treatment or control of solid tumors, such as, for example, breast, colon, lung and prostate tumors. These may be ised in

The present invention is also directed to a pharmaceutical compositon which comprises a compound of formula I, or a pharmaceutically acceptable salt or ester thereof, and a pharmaceutically acceptable carrier. These may be used in a method of treating a disease based on the interaction of MDM2 protein with a p53-like peptide comprising administering to a patient in need of such treatment a therapeutically effective amount of at least one compound of formula I or a pharmaceutically acceptable salt or ester thereof.

The compounds of the present invention can be prepared according to the following schemes. The following definitions are provided as applicable to the synthesis schemes:

### Synthesis

The compounds of formula I can be prepared according to the scheme 1.

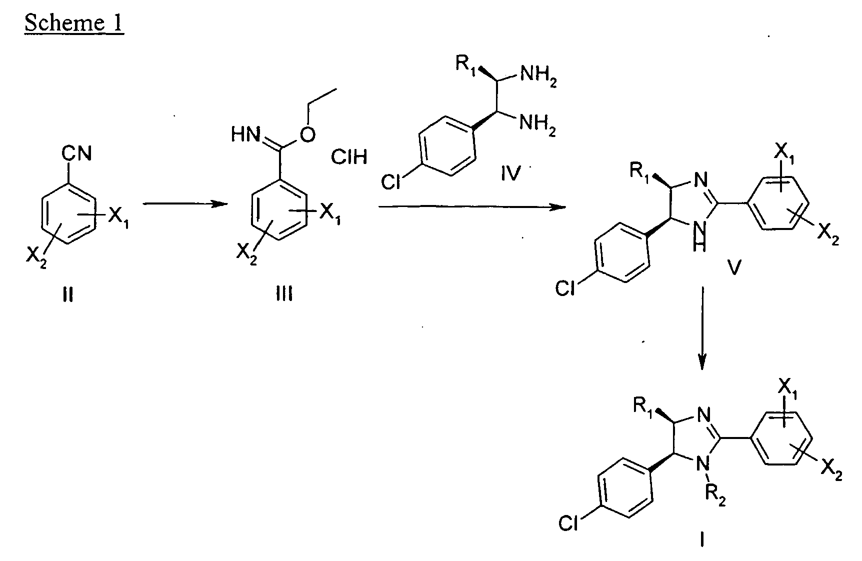

Many benzonitriles of formula II are commercially available. They are converted to the imidate salts (III) using HCl gas in ethanol. The rate of the reaction depends on the substituents on the phenyl ring. In cases where X₁ or X₂ ≠ H, it may be necessary to run the reaction under pressure of HCl over a longer period of time. Condensation of the imidates (III) with the 1,2-diamines (IV) is carried out in ethanol at 40-100 °C in the presence or absence of a base such as triethylamine.

If it is desired to prepare the compounds of formula II which are not commercially available, many synthetic methods known in the art can be employed. Suitable processes for synthesizing these benzonitriles are provided in the examples. Following schemes illustrate some of these methods.

A compound of formula B (V¹⁶ can be any suitable group such as V¹, V², V³, V⁴, or V⁵) can be prepared by alkylation of a compound of formula A with V⁶X (X = Cl, Br, I) using conventional methods (scheme V). The phenoxide anion is generated by a base such as cesium carbonate or potassium carbonate. The reaction typically is carried out in refluxing acetone. V⁶ can also be introduced using Mitsunobu reaction (see for example, Hughes, D. L. Org. React. 1992, 42, 335-656).

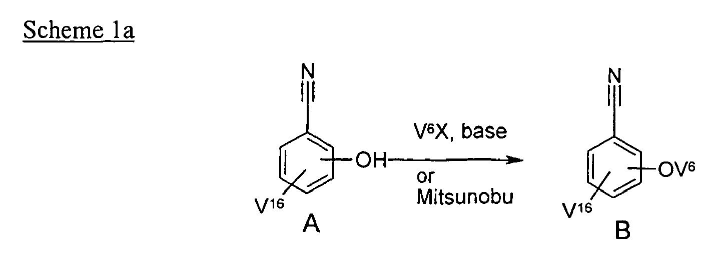

A compound of formula C (V¹⁶ can be any suitable group such as V¹, V², V³, V⁴, or V⁵) can be converted into the benzonitrile D using literature procedures (Karmarkar, S. N; Kelkar, S. L.; Wadia, M. S. Synthesis 1985, 510-512; Bergeron, R. J. et al. J. Med. Chem. 1999, 42, 95-108). V group can then be introduced using V⁶X (X = Cl, Br, I) or Mitsunobu reaction to give the benzonitrile 13 (scheme 1b).

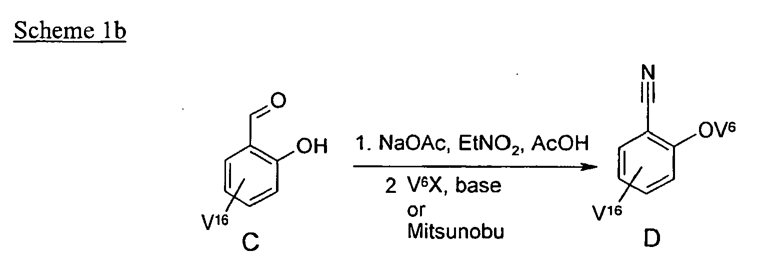

A compound of formula F can be prepared by bromination or iodination of phenol E (Scheme VII), (V¹⁶ can be any suitable group such as V¹, V², V³, V⁴, or V⁵). Reaction conditions such as *N*-bromosuccinamide/tetrahydrofuran or iodine/thallium(I) acetate can be utilized (see for example, Carreno, M. C.; Garcia Ruano, J. L.; Sanz, G.; Toledo, M. A.; Urbano, A. Synlett 1997, 1241-1242; Cambie, R. C.; Rutledge, P. S.; Smith-Palmer, T.; Woodgate, P. D. J. Chem. Soc., Perkin Trans. 1 1976, 1161-4). V⁵ group can then be introduced using V⁶X (X = Cl, Br, I) or Mitsunobu reaction. Methods of converting aromatic halides to the corresponding nitriles are known in the art (see for example, Okano, T.; Iwahara, M.; Kiji, J., Synlett 1998, 243). Cyanation of the halide (X' = Br, I) can be accomplished using zinc cyanide with a catalyst such as tetrakis(triphenylphosphine)palladium (0). Solvents such dimethylformamide can be used and the reaction temperature is between 80-110 C.

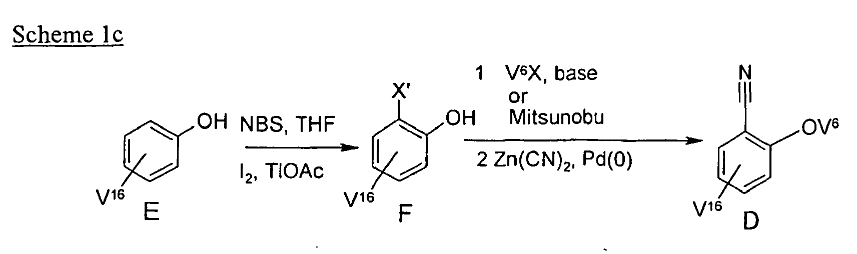

In scheme ID, amination of aromatic halide G using HNV⁷V⁸ and palladium catalyst can be utilized to provide the benzonitrile of formula H (see for example, Harris, M. C.; Geis, O.; Buchwald, S. L. J. Org. Chem. 1999, 64, 6019).

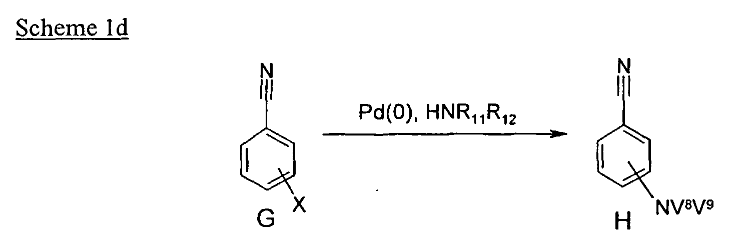

A compound of formula D (V¹⁶ can be any suitable group such as V¹, V², V³, V⁴, or V⁵) can be prepared by nucleophilic substitution of 2-halobenzonitrile I (scheme 1e). (see for example, X = F: Wells, K. M.; Shi, Y.-J.; Lynch, J. E.; Humphrey, G. R.; Volante, R. P.; Reider, P. J. Tetrahedron Lett. 1996, 37, 6439-6442; X = NO₂: Harrison, C. R.; Lett, R. M.; McCann, S. F.; Shapiro, R.; Stevenson, T. M. WO 92/03421, 1992).

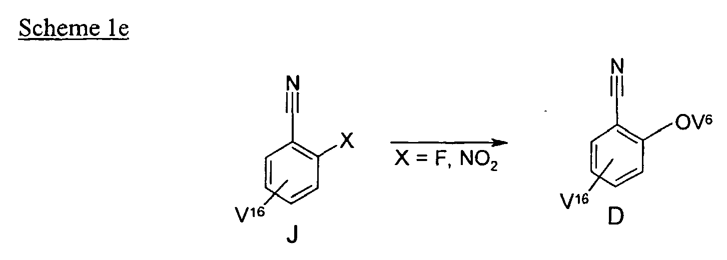

To prepare the benzonitrile of formula L wherein V¹, V², V³, V⁴, or V⁵ = OV⁶, sequential alkylation of the diol 19 with suitable V⁶X (X = Cl, Br, I) are used. The bromides K are then converted to the nitriles L using zinc cyanide and Pd(0) catalyst (scheme If).

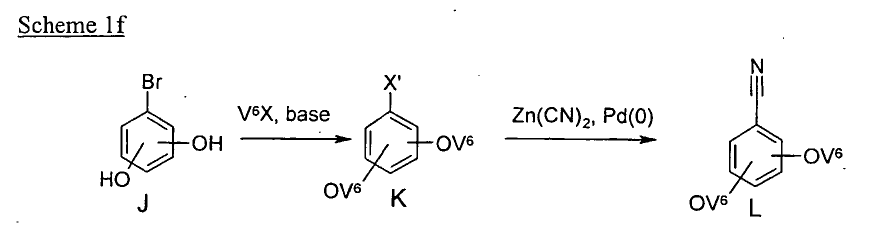

The 1,2-diamines of formula IV are prepared using procedures as reported by Shatzmiller, S.; Bercovici, S. Liebigs Ann. Chem. 1992, 1005-1009 (Scheme 2). The ketone VI is converted to the corresponding oxime ether. α-Bromination of the oxime ether with N-bromosuccinimide and reaction of the α-bromo oxime ether with ammonia in methanol gives the α-amino oxime ether VII. Reduction of VII with lithium aluminum hydride gives a mixture of cis and trans 1,2-diamine (~4-6:1 ratio of cis:trans). The crude 1,2-diamine is then reacted with compound of formula III to give a mixture of cis and trans products. The desired compound (formula V) can be isolated from the mixture by preparative chromatography techniques.

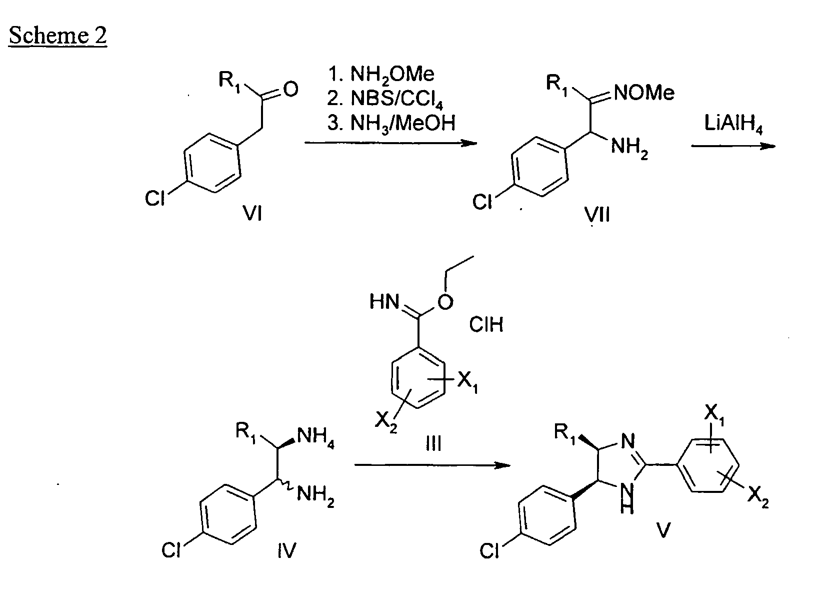

When R₂ = COR₁₁, the compound V is converted to the compound of formula VIII using a compound of formula CICOR₁₁ (a known compound or a compound prepared by known methods) in the presence of a base such triethylamine (Scheme 3).

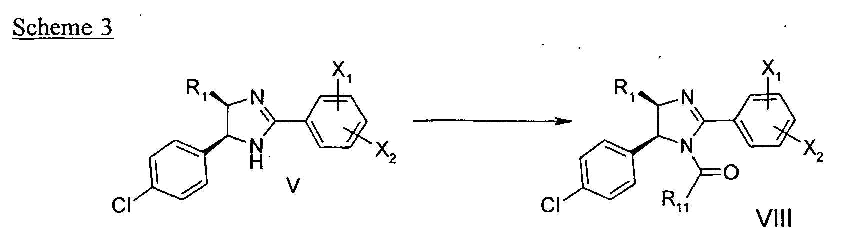

When R₂ = CONR₁₂R₁₃, the compound of formula V is reacted with phosgene at 0 °C in the presence of a base such as triethylamine followed by the treatment of a compound of formula NHR₁₂R₁₃ (a known compound or a compound prepared by known methods) to give the compound of formula IX (Scheme 4).

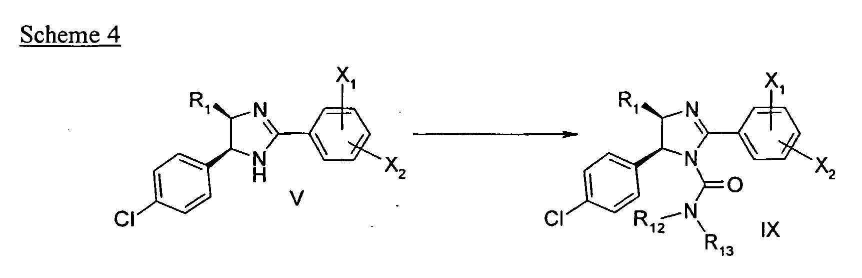

The present invention encompasses the following Examples. Structural formulas follow. With regard to structural formulas, it is understood that oxygen and nitrogen atoms with available electrons have a hydrogen bound thereto, as indicated by compound name.

The following compounds were tested according to the assay described below and exhibited IC₅₀'s from about 0.5 µM to about 300µM.

### Example 1

### Ethyl 2-ethoxy-4-methoxy-benzimidate hydrochloride

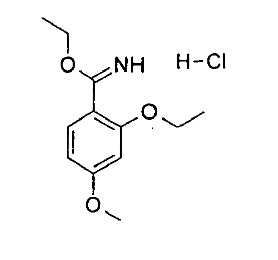

A mixture of the 2-hydroxy-4-methoxybenzaldehyde (20 g, 128.8 mmol), sodium acetate (35.05 g, 257.6 mmol) and nitroethane (19 mL, 257.6 mmol) in glacial acetic acid (100 mL) was heated at gentle reflux for 12 h. The reaction mixture was then poured into ~1000 mL of ice water (1:1 ratio of ice and water). The product was extracted with ethyl acetate (3 x 200 mL). The organic extracts were washed with sodium bicarbonate solution until the aqueous layer had pH ~8. The organic layers were then dried over anhydrous magnesium sulfate and concentrated *in vacuo* to afford 2-hydroxy-4-methoxy-benzonitrile as a yellow oil (16.5 g, 86%). It was used without further purification.

To a solution of 2-hydroxy-4-methoxy-benzonitrile (9.637 g, 64.61 mmol) in ethanol (50 mL) were added potassium carbonate (17.88 g, 129.2 mmol) and iodoethane (15.7 mL, 193.8 mmol). The reaction mixture was heated at gentle reflux for 12 h. The solvent was removed to afford a yellow-brown paste. It was then taken in diethyl ether (50 mL) and water (20 mL). The layers were separated and the aqueous layer was extracted with diethyl ether (2 x 150 mL). The combined organic extracts were washed with water (1 x 20 mL), brine (1 x 20 mL), and dried over anhydrous sodium sulfate. The solids were then filtered off, and the filtrate was concentrated *in vacuo.* Purification of the crude residue by flash chromatography (Biotage system, KP-Sil™ 32-63 µm, 60 Å silica gel) eluting with 10-15% ethyl acetate in hexanes yielded 2-ethoxy-4-methoxy-benzonitrile as yellow solids (9.487 g, 83%).

Hydrogen chloride gas was passed through a solution of 2-ethoxy-4-methoxy-benzonitrile (6.3 g, 35.55 mmol) in anhydrous ethanol (70 mL) cooled to -10 °C. After 30 min, hydrogen chloride gas was stopped and the reaction mixture was stirred at room temperature in a closed reaction vessel for 4 d. The reaction vessel was cooled to 0 °C before the stopper was removed. Argon gas was passed through the solution to remove excess hydrogen chloride gas. The solvent was evaporated and the residue was triturated in diethyl ether (100 mL) to afford ethyl 2-ethoxy-4-methoxy-benzimidate hydrochloride (7.3 g, 79%). It was used without further purification.

### Example 2

The following compounds were prepared in a manner analogous to that described in Example 1:
(a) Ethyl 2-isopropoxy-4-methoxy-benzimidate hydrochloride from 2-hydroxy-4-methoxy-benzonitrile and isopropyl iodide. 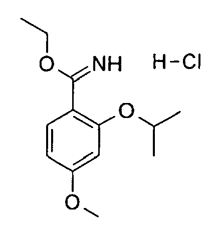
(b) Ethyl 2-(2-fluoro-ethoxy)-4-methoxy-benzimidate hydrochloride from 2-hydroxy-4-methoxy-benzonitrile and 1-bromo-2-fluoro-ethane. 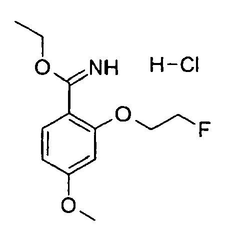
(c) Ethyl 2,4-diethoxy-benzimidate hydrochloride from 2,4-diethoxy-benzaldehyde.

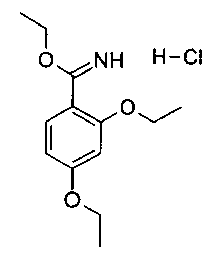

### Example 3

### 1-(4-Chloro-phenyl)-3-cyclopentyl-propan-1-one

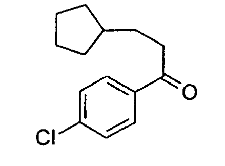

Aluminum chloride (4.979 g, 37.34 mmol) was added in small portions to a solution of 3-cyclopentylpropionyl chloride (3 g, 18.67 mmol) in 1,2-dichloroethane (100 mL) cooled to 0 °C. After 15 min., chlorobenzene (6.304 g, 56.01 mmol) was added. The reaction mixture was stirred at 0 °C for 1 h and at room temperature for 48 h. It was then poured into a mixture of ice and water. The product was extracted with ethyl acetate. The organic layers were washed with saturated solution of sodium bicarbonate (1 x 30 mL), brine (1 x 20 mL) and dried over anhydrous magnesium sulfate. The solids were then filtered off, and the filtrate was concentrated *in vacuo.* Purification of the crude residue by flash chromatography (Biotage system, KP-Sil™ 32-63 µm, 60 Å silica gel) eluting with hexanes yielded 1-(4-chloro-phenyl)-3-cyclopentyl-propan-1-one (1.12 g, 25%) as yellow oil.

### Example 4

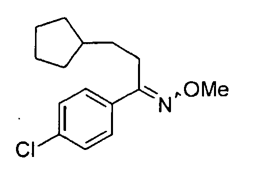

### 1-(4-Chloro-phenyl)-3-cyclopentyl-propan-1-one O-methyl-oxime

To a solution of 1-(4-chloro-phenyl)-3-cyclopentyl-propan-1-one (1.12 g, 4.731 mmol) in ethanol (15 mL) was added potassium carbonate (1.962 g, 14.19 mmol) and methoxyamine hydrochloride (494 mg, 5.914 mmol). The reaction mixture was heated at reflux for 12 h. Upon cooling to room temperature, the reaction mixture was filtered, and the white solids were washed with diethyl ether. The filtrate was concentrated *in vacuo,* and the residue was partitioned between diethyl ether and water. The product was extracted with diethyl ether (2 x 30 mL). The organic layers were washed with brine and dried over anhydrous sodium sulfate. The solids were then filtered off, and the filtrate was concentrated *in vacuo.* Purification of the crude residue by flash chromatography (Biotage system, KP-Sil™ 32-63 µm, 60 Å silica gel) eluting with 1-2% ethyl acetate in hexanes yielded 1-(4-chloro-phenyl)-3-cyclopentyl-propan-1-one O-methyl-oxime (762 mg, 61 %) as clear oil.

### Example 5

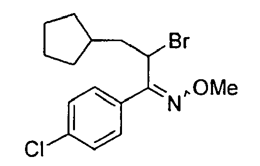

### 2-Bromo-1-(4-chloro-phenyl)-3-cyclopentyl-propan-1-one O-methyl-oxime

To a solution of 1-(4-chloro-phenyl)-3-cyclopentyl-propan-1-one O-methyl-oxime (1.120 g, 4.214 mmol) in carbon tetrachloride were added N-bromosuccinimide (812 mg, 4.425 mmol) and benzoyl peroxide (102 mg, 0.4 mmol). The resulting mixture was heated at reflux for 12 h. Upon cooling to room temperature, the solids were filtered and washed with diethyl ether. The filtrate was washed with aqueous solution of sodium bicarbonate, sodium thiosulfate and brine. It was then dried with anhydrous sodium sulfate and concentrated *in vacuo.* Purification of the crude residue by flash chromatography (Biotage system, KP-Sil™ 32-63 µm, 60 Å silica gel) eluting with 1-2% ethyl acetate in hexanes yielded 2-bromo-1-(4-chloro-phenyl)-3-cyclopentyl-propan-1-one O-methyl-oxime (1.16 g, 80%) as yellow oil.

### Example 6

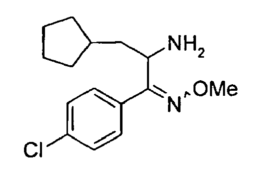

### 2-Amino-1-(4-chloro-phenyl)-3-cyclopentyl-propan-1-one O-methyl-oxime

The 2-bromo-1-(4-chloro-phenyl)-3-cyclopentyl-propan-1-one O-methyl-oxime (1.16 g, 2.901 mmol) was dissolved in a solution of ammonia in methanol (30 mL, ~7 N). The reaction flask was sealed with a Teflon stopper, and the reaction mixture was stirred at 55-60 °C for 2 d. It was cooled to 0 °C then the stopper was removed. The reaction mixture was concentrated to remove ammonia and methanol. The residue was partitioned between water and diethyl ether. The product was extracted with diethyl ether (2 x 20 mL). The organic layers were washed with brine, dried over anhydrous sodium sulfate and concentrated *in vacuo.* Purification of the crude residue by flash chromatography (Biotage system, KP-Sil™ 32-63 µm, 60 Å silica gel) eluting with 50-100% ethyl acetate + 0.1% triethylamine in hexanes yielded 2-amino-1-(4-chlorophenyl)-3-cyclopentyl-propan-1-one O-methyl-oxime (423 mg, 45%) as yellow oil.

### Example 7

### 1-(4-Chloro-phenyl)-3-cyclopentyl-propane-1,2-diamine

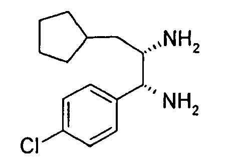

A solution of 2-arnino-1-(4-chloro-phenyl)-3-cyclopentyl-propan-1-one O-methyl-oxime (420 mg, 1.567 mmol) in diethyl ether (3 mL) was added dropwise to a slurry mixture of lithium aluminumhydride (307 mg, 7.835 mmol) in diethyl ether (30 mL) cooled to 0 °C. At the end of addition, the icebath was removed and the reaction was stirred at room temperature for 3 h. The reaction mixture was cooled to 0 °C and aqueous solution of sodium chloride was added to quench the excess lithium aluminumhydride. Sodium hydroxide solution was added and the biphasic mixture was stirred for 1 h. The product was extracted with diethyl ether (3 x 30 mL). The ethereal extracts were washed with brine and dried over anhydrous sodium sulfate. Evaporation of the solvent gave 1-(4-chloro-phenyl)-3-cyc-lopentyl-propane-1,2-diamine as yellow oil (362.7 mg, 96%, 4.3:1.0 ratio of cisarans). The crude product was used without further purification.

### Example 8

The following compounds were prepared in a manner analogous to that described in Examples 3-7:
a. 1-(4-Chloro-phenyl)-propane-1,2-diamine 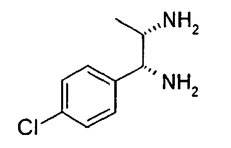
b. 1-(4-Chloro-phenyl)-butane-1,2-diamine 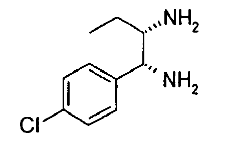
c. 1-(4-Chloro-phenyl)-pentane-1,2-diamine 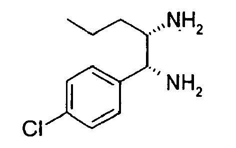
d. 1-(4-Chloro-phenyl)-hexane-1,2-diamine 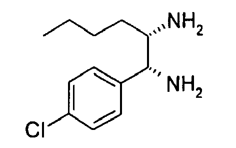
e. 1-(4-Chloro-phenyl)-heptane-1,2-diamine 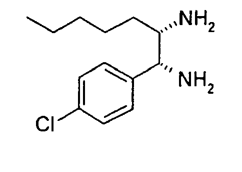
f. 1-(4-Chloro-phenyl)-4-methyl-pentane-1,2-diamine 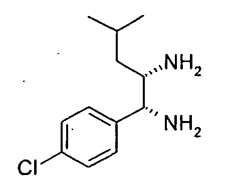
g. 1-(4-Chloro-phenyl)-5-methyl-hexane-1,2-diamine 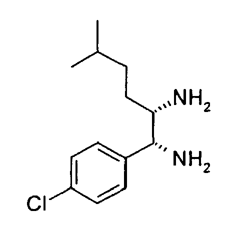
h. 1-(4-Chloro-phenyl)-2-cyclopentyl-ethane-1,2-diamine 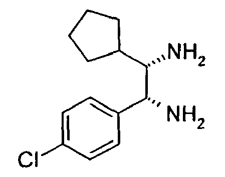
i. 1-(4-Chloro-phenyl)-2-cyclohexyl-ethane-1,2-diamine 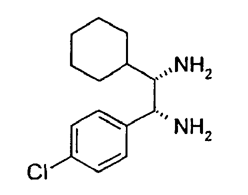
j. 1-(4-Chloro-phenyl)-3-cyclohexyl-propane-1,2-diamine 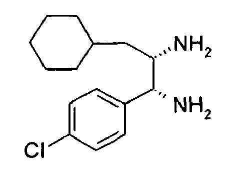
k. 1-(4-Chloro-phenyl)-4-cyclohexyl-butane-1,2-diamine

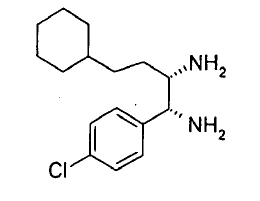

### Example 9

### 5-(4-chloro-phenyl)-4-cyclopentylmethyl-2-(2-ethoxy-4-methoxy-phenyl)-4,5-dihydro-1H-imidazole

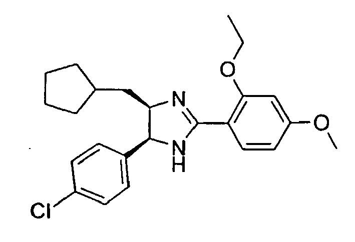

To a solution of 1-(4-chloro-phenyl)-3-cyclopentyl-propane-1,2-diamine (170 mg, 0.672 mmol) and ethyl 2-ethoxy-4-methoxy-benzimidate hydrochloride (210 mg, 0.806 mmol) in ethanol (10 mL) was added triethylamine (82 µL, 0.806 mmol). The reaction mixture was heated at gentle reflux for 6 h. The solvent was removed and the residue was partitioned between water and methylene chloride. The product was extracted with methylene chloride (2 x 20 mL). The organic layers were washed with brine, dried over anhydrous sodium sulfate and concentrated *in vacuo.* The residue was purified by flash chromatography (Biotage system, KP-Sil™ 32-63 µm, 60 Å silica gel) eluting with ethyl acetate then 5-10% methanol in ethyl acetate to give 5-(4-chloro-phenyl)-4-cyclopentylmethyl-2-(2-ethoxy-4-methoxy-phenyl)-4,5-dihydro-1H-imidazole (174 mg, 63%, 4.5:1.0 ratio of cis:trans) as a white foam. HR-MS (ES, *m*/*z*) observed 413.1993, calculated for C₂₄H₃₀N₂O₂Cl [(M+H)⁺] 413.1991.

### Example 10

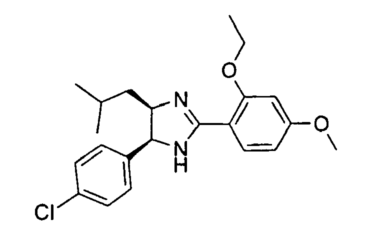

5-(4-Chloro-phenyl)-2-(2-ethoxy-4-methoxy-phenyl)-4-isobutyl-4,5-dihydro-1H-imidazole was prepared from 1-(4-chloro-phenyl)-4-methyl-pentane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 387.1837, calculated for C₂₂H₂₈N₂O₂Cl [(M+H)⁺] 387.1834.

### Example 11

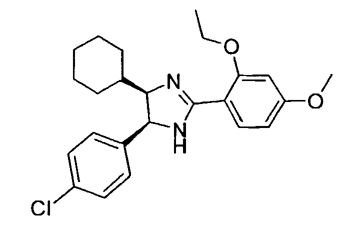

5-(4-Chloro-phenyl)-4-cyclohexyl-2-(2-ethoxy-4-methoxy-phenyl)-4,5-dihydro-1H-imidazole was prepared from 1-(4-chloro-phenyl)-2-cyclohexyl-ethane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 413.1993, calculated for C₂₄H₃₀N₂O₂Cl [(M+H)⁺] 413.1991.

### Example 12

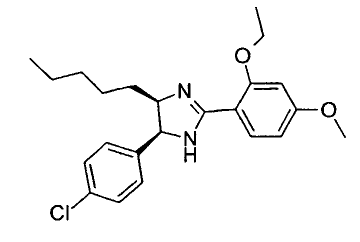

5-(4-Chloro-phenyl)-2-(2-ethoxy-4-methoxy-phenyl)-4-pentyl-4,5-dihydro-1H-imidazole was prepared from 1-(4-chloro-phenyl)-heptane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxyphenyl)-4,5-dihydro-1H-imidazole (Example 9). HR-MS (ES, *m*/*z*) observed 401.1993, calculated for C₂₃H₃₀N₂O₂Cl [(M+H)⁺] 401.1991.

### Example 13

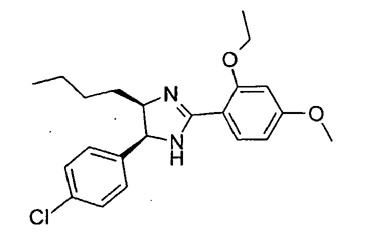

4-Butyl-5-(4-chloro-phenyl)-2-(2-ethoxy-4-methoxy-phenyl)-4,5-dihydro-1H-imidazole was prepared from 1-(4-chloro-phenyl)-hexane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxyphenyl)-4,5-dihydro-1H-imidazole (Example 9). HR-MS (ES, *m*/*z*) observed 387.1839, calculated for C₂₂H₂₈N₂O₂Cl [(M+H)⁺] 387.1834.

### Example 14

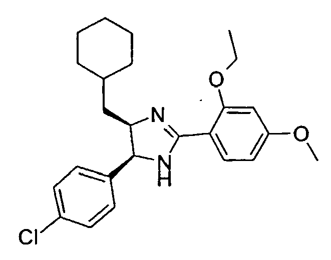

5-(4-Chloro-phenyl)-4-cyclohexylmethyl-2-(2-ethoxy-4-methoxy-phenyl)-4,5-dihydro-1H-imidazole was prepared from 1-(4-chloro-phenyl)-3-cyclohexyl-propane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 427.2150, calculated for C₂₅H₃₂N₂O₂Cl [(M+H)⁺]427.2147.

### Example 15

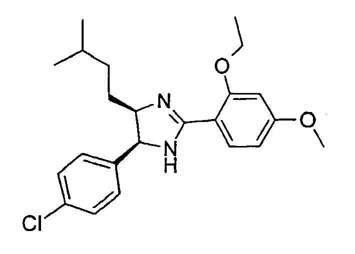

5-(4-Chloro-phenyl)-2-(2-ethoxy-4-methoxy-phenyl)-4-(3-methyl-butyl)-4,5-dihydro-1H-imidazole was prepared from 1-(4-chloro-phenyl)-5-methyl-hexane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 401.1994, calculated for C₂₃H₃₀N₂O₂Cl [(M+H)⁺] 401.1991.

### Example 16

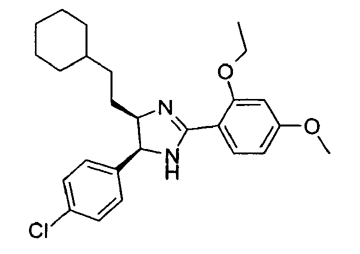

5-(4-Chloro-phenyl)-4-(2-cyclohexyl-ethyl)-2-(2-ethoxy-4-methoxy-phenyl)-4,5-dihydro-1H-imidazole was prepared from 1-(4-chloro-phenyl)-4-cyclohexyl-butane-1,2-diamine and ethyl 2-ethoxy-4-methoxy-benzimidate hydrochloride in an analogous manner as described for the preparation of 5-(4-chloro-pheriyl)-4-cyclopentylmethyl-2-(2-ethoxy-4-methoxy-phenyl)-4,5-dihydro-1H-imidazole (Example 9). HR-MS (ES, *m*/*z*) observed 441.2309, calculated for C₂₆H₃₄N₂O₂Cl [(M+H)⁺] 441.2304.

### Example 17

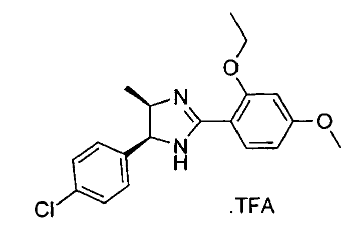

5-(4-Chloro-phenyl)-2-(2-ethoxy-4-methoxy-phenyl)-4-methyl-4,5-dihydro-1H-imidazole, trifluoroacetate salt was prepared from 1-(4-chloro-phenyl)-propane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 345.1366, calculated for C₁₉H₂₂N₂O₂Cl [(M+H)⁺] 345.1365.

### Example 18

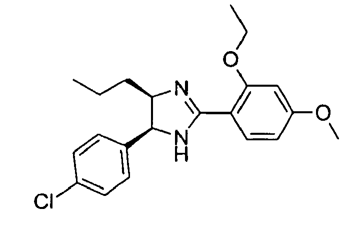

5-(4-Chloro-phenyl)-2-(2-ethoxy-4-methoxy-phenyl)-4-propyl-4,5-dihydro-1H-imidazole was prepared from 1-(4-chloro-phenyl)-pentane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxyphenyl)-4,5-dihydro-1H-imidazole (Example 9). HR-MS (ES, *m*/*z*) observed 373.1680, calculated for C₂₁H₂₆N₂O₂Cl [(M+H)⁺] 373.1678.

### Example 19

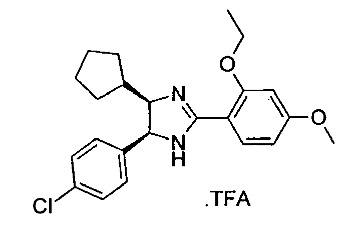

5-(4-Chloro-phenyl)-4-cyclopentyl-2-(2-ethoxy-4-methoxy-phenyl)-4,5-dihydro-1H-imidazole, trifluoroacetate salt was prepared from 1-(4-chloro-phenyl)-2-cyclopentyl-ethane-1,2-diamine and ethyl 2-ethoxy-4-methoxy-benzimidate hydrochloride in an analogous manner as described for the preparation of 5-(4-chlorophenyl)-4-cyclopentylmethyl-2-(2-ethoxy-4-methoxy-phenyl)-4,5-dihydro-1H-imidazole (Example 9). HR-MS (ES, *m*/*z*) observed 399.1834, calculated for C₂₃H₂₈N₂O₂Cl [(M+H)⁺] 399.1834.

### Example 20

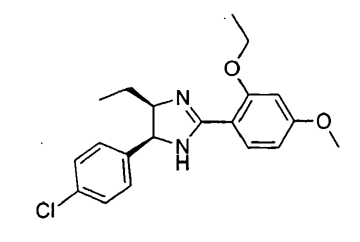

5-(4-Chloro-phenyl)-2-(2-ethoxy-4-methoxy-phenyl)-4-ethyl-4,5-dihydro-1H-imidazole was prepared from 1-(4-chloro-phenyl)-butane-1,2-diamine and ethyl 2-ethoxy-4-methoxy-benzimidate hydrochloride in an analogous manner as described for the preparation of 5-(4-chloro-phenyl)-4-cyclopentylmethyl-2-(2-ethoxy-4-methoxyphenyl)-4,5-dihydro-1H-imidazole (Example 9). HR-MS (ES, *m*/*z*) observed 359.1524, calculated for C₂₀H₂₄N₂O₂Cl [(M+H)⁺] 359.1521.

### Example 21

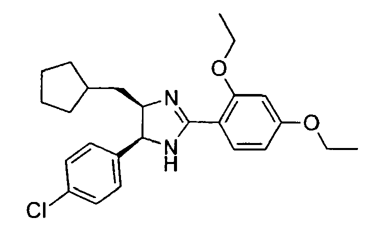

5-(4-Chloro-phenyl)-4-cyclopentylmethyl-2-(2,4-diethoxy-phenyl)-4,5-dihydro-1H-imidazole was prepared from 1-(4-chloro-phenyl)-3-cyclopentyl-propane-1,2-diamine and ethyl 2,4-diethoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 427.2152, calculated for C₂₅H₃₂N₂O₂Cl [(M+H)⁺] 427.2147.

### Example 22

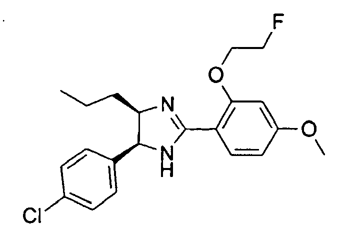

5-(4-Chloro-phenyl)-2-[2-(2-fluoro-ethoxy)-4-methoxy-phenyl]-4-propyl-4,5-dihydro-1H-imidazole was prepared from 1-(4-chloro-phenyl)-pentane-1,2-diamine and ethyl 2-(2-fluoro-ethoxy)-4-methoxy-benzimidate hydrochloride in an analogous manner as described for the preparation of 5-(4-chloro-phenyl)-4-cyclopentylmethyl-2-(2-ethoxy-4-methoxy-phenyl)-4,5-dihydro-1H-imidazole (Example 9). HR-MS (ES, *m*/*z*) observed 391.1587, calculated for C₂₁H₂₅N₂O₂FCl [(M+H)⁺] 391.1583.

### Example 23

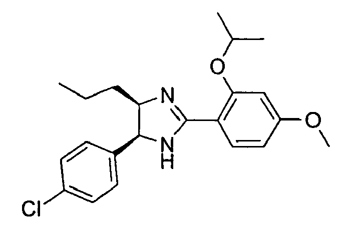

5-(4-Chloro-phenyl)-2-(2-isopropoxy-4-methoxy-phenyl)-4-propyl-4,5-dihydro-1H-imidazole was prepared from 1-(4-chloro-phenyl)-pentane-1,2-diamine and ethyl 2-isopropoxy-4-methoxy-benzimidate hydrochloride in an analogous manner as described for the preparation of 5-(4-chloro-phenyl)-4-cyclopentylmethyl-2-(2-ethoxy-4-methoxyphenyl)-4,5-dihydro-1H-imidazole (Example 9). HR-MS (ES, *m*/*z*) observed 387.1840, calculated for C₂₂H₂₈N₂O₂Cl [(M+H)⁺] 387.1834.

### Example 24

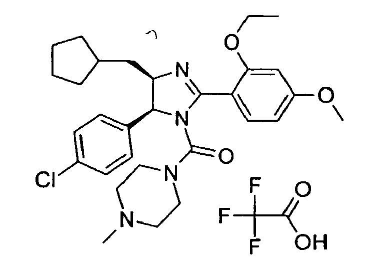

### [5-(4-Chloro-phenyl)-4-cyclopentylmethyl-2-(2-ethoxy-4-methoxy-phenyl)-4,5-dihydro-imidazol-1-yl]-(4-methyl-piperazin-1-yl)-methanone, trifluoroacetate salt

To a solution of 5-(4-chloro-phenyl)-4-cyclopentylmethyl-2-(2-ethoxy-4-methoxyphenyl)-4,5-dihydro-1H-imidazole (160 mg, 0.387 mmol) in methylene chloride (15 mL) cooled to 0 °C were sequentially added triethylamine (108 µL, 0.774 mmol) and phosgene (589 µL, 1.161 mmol, 1.97 M in toluene). The reaction mixture was stirred at 0 °C under Argon for 30 min. The solvent and excess reagents were removed by rotovap, and the residue was purified by flash chromatography (Biotage system, KP-Sil™ 32-63 µm, 60 Å silica gel) eluting with 20-25% ethyl acetate in hexanes to give 5-(4-chloro-phenyl)-4-cyclopentylmethyl-2-(2-ethoxy-4-methoxy-phenyl)-4,5-dihydroimidazole-1-carbonyl chloride (132.6 mg, 72%) as white solids.

A solution of 5-(4-chloro-phenyl)-4-cyclopentylmethyl-2-(2-ethoxy-4-methoxyphenyl)-4,5-dihydro-imidazole-1-carbonyl chloride (50 mg, 0.105 mmol) in methylene chloride (2 mL) was added to a stirred mixture of 1-methylpiperazine (18 µL, 0.158 mmol) and triethylamine (29 µL, 0.210 mmol) in methylene chloride (3 mL) cooled to 0 °C. The reaction mixture was stirred at room temperature for 30 min then concentrated *in vacuo.* Purification of the crude residue by preparative HPLC (Zorbax C₁₈) gave [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (64 mg, 93%, white solids) as trifluoroacetic acid salt. HR-MS (ES, *m*/*z*) observed 539.2789, calculated for C₃₀H₄₀N₄O₃Cl [(M+H)⁺] 539.2784.

### Example 25

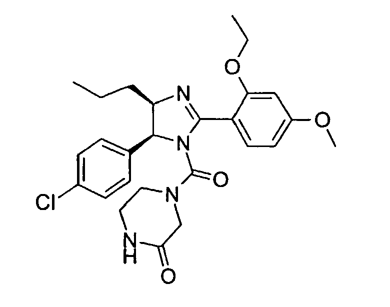

4-[5-(4-Chloro-phenyl)-2-(2-ethoxy-4-methoxy-phenyl)-4-propyl-4,5-dihydroimidazole-1-carbonyl]-piperazin-2-one was prepared from 5-(4-chloro-phenyl)-2-(2-ethoxy-4-methoxy-phenyl)-4-propyl-4,5-dihydro-1H-imidazole (Example 18) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 499.2113, calculated for C₂₆H₃₂N₄O₄Cl [(M+H)⁺] 499.2107.

### Example 26

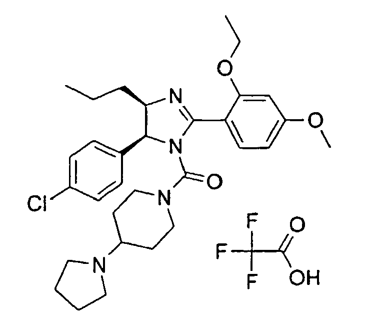

[5-(4-Chloro-phenyl)-2-(2-ethoxy-4-methoxy-phenyl)-4-propyl-4,5-dihydro-imidazol-1-yl]-(4-pyrrolidin-1-yl-piperidin-1-yl)-methanone, trifluoroacetate salt was prepared from 5-(4-chloro-phenyl)-2-(2-ethoxy-4-methoxy-phenyl)-4-propyl-4,5-dihydro-1H-imidazole (Example 18) in an analogous manner as described for the preparation of [5-(4-chloro-phenyl)-4-cyclopentylmethyl-2-(2-ethoxy-4-methoxyphenyl)-4,5-dihydro-imidazol-l-yl]-(4-methyl-piperazin-1-yl)-methanone (Example 24). HR-MS (ES, *m*/*z*) observed 553.2940, calculated for C₃₁H₄₂N₄O₃Cl [(M+H)⁺] 553.2940.

### Example 27

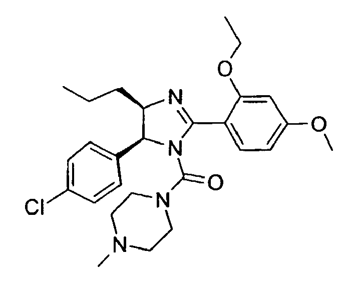

[5-(4-Chloro-phenyl)-2-(2-ethoxy-4-methoxy-phenyl)-4-propyl-4,5-dihydro-imidazol-1-yl]-(4-methyl-piperazin-1-yl)-methanone was prepared from 5-(4-chlorophenyl)-2-(2-ethoxy-4-methoxy-phenyl)-4-propyl-4,5-dihydro-1H-imidazole (Example 18) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 499.2477, calculated for C₂₇H₃₆N₄O₃Cl [(M+H)⁺] 499.2471.

### Example 28

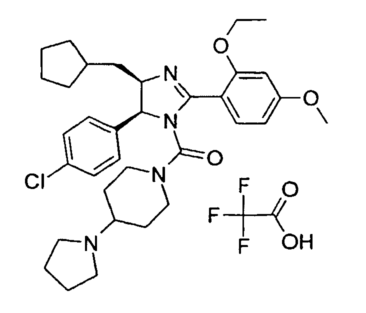

[5-(4-Chloro-phenyl)-4-cyclopentylmethyl-2-(2-ethoxy-4-methoxy-phenyl)-4,5-dihydro-imidazol-1-yl]-(4-pyrrolidin-1-yl-piperidin-1-yl)-methanone, trifluoroacetate salt was prepared from 5-(4-chloro-phenyl)-4-cyclopentylmethyl-2-(2-ethoxy-4-methoxy-phenyl)-4,5-dihydro-1H-imidazole (Example 9) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 593.3257, calculated for C₃₄H₃₅N₄O₃Cl [(M+H)⁺] 593.3253.

### Example 29

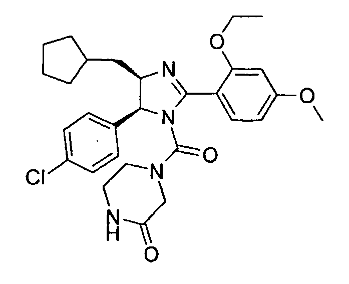

4-[5-(4-Chloro-phenyl)-4-cyclopentylmethyl-2-(2-ethoxy-4-methoxy-phenyl)-4,5-dihydro-imidazole-1-carbonyl]-piperazin-2-one was prepared from 5-(4-chloro-phenyl)-4-cyclopentylmethyl-2-(2-ethoxy-4-methoxy-phenyl)-4,5-dihydro-1H-imidazole (Example 9) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 539.2423, calculated for C₂₉H₃₆N₄O₄Cl [(M+H)⁺] 539.2420.

### Example 30

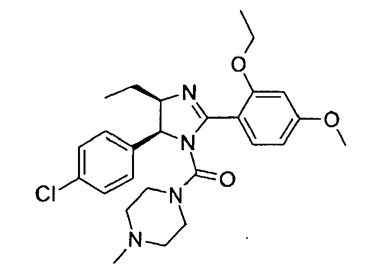

[5-(4-Chloro-phenyl)-2-(2-ethoxy-4-methoxy-phenyl)-4-ethyl-4,5-dihydro-imidazol-1-yl]-(4-methyl-piperazin-1-yl)-methanone was prepared from 5-(4-chlorophenyl)-2-(2-ethoxy-4-methoxy-phenyl)-4-ethyl-4,5-dihydro-1H-imidazole (Example 20) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 485.2323, calculated for C₂₆H₃₄N₄O₃Cl [(M+H)⁺] 485.2314.

### Example 31

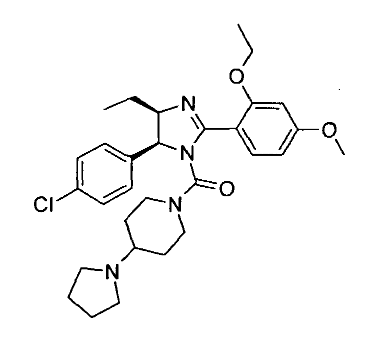

[5-(4-Chloro-phenyl)-2-(2-ethoxy-4-methoxy-phenyl)-4-ethyl-4,5-dihydro-imidazol-1-yl]-(4-pyrrolidin-1-yl-piperidin-1-yl)-methanone was prepared from 5-(4-chloro-phenyl)-2-(2-ethoxy-4-methoxy-phenyl)-4-ethyl-4,5-dihydro-1H-imidazole (Example 20) in an analogous manner as described for the preparation of [5-(4-chlorophenyl)-4-cyclopentylmethyl-2-(2-ethoxy-4-methoxy-phenyl)-4,5-dihydro-imideol-1-yl]-(4-methyl-piperazin-1-yl)-methanone (Example 24). HR-MS (ES, *m*/*z*) observed 539.2792, calculated for C₃₀H₄₀N₄O₃Cl [(M+H)⁺] 539.2784.

### Example 32

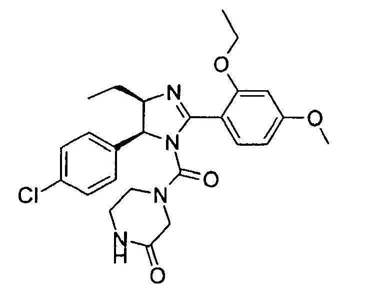

4-[5-(4-Chloro-phenyl)-2-(2-ethoxy-4-methoxy-phenyl)-4-ethyl-4,5-dihydroimidazole-1-carbonyl]-piperazin-2-one was prepared from 5-(4-chloro-phenyl)-2-(2-ethoxy-4-methoxy-phenyl)-4-ethyl-4,5-dihydro-1H-imidazole (Example 20) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 485.1959, calculated for C₂₅H₃₀N₄O₃Cl [(M+H)⁺] 485.1950.

### Example 33

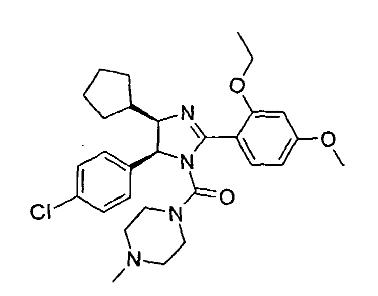

[5-(4-Chloro-phenyl)-4-cyclopentyl-2-(2-ethoxy-4-methoxy-phenyl)-4,5-dihydro-imidazol-1-yl]-(4-methyl-piperazin-1-yl)-methanone was prepared from 5-(4-chlorophenyl)-4-cyclopentyl-2-(2-ethoxy-4-methoxy-phenyl)-4,5-dihydry-1H-imidazole, trifluoroacetate salt (Example 19) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 525.2634, calculated for C₂₉H₃₈N₄O₃Cl [(M+H)⁺] 525.2627.

### Example 34

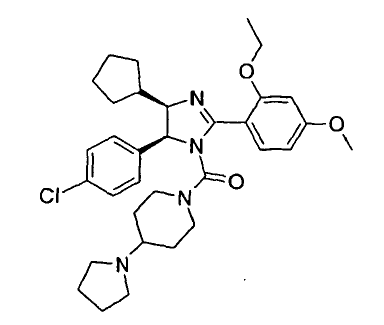

[5-(4-Chloro-phenyl)-4-cyclopentyl-2-(2-ethoxy-4-methoxy-phenyl)-4,5-dihydro-imideol-1-yl]-(4-pyrrolidin-1-yl-piperidin-1-yl)-methanone was prepared from 5-(4-chloro-phenyl)-4-cyclopentyl-2-(2-ethoxy-4-methoxy-phenyl)-4,5-dihydro-1H-imidazole, trifluoroacetate salt (Example 19) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 579.3104, calculated for C₃₃H₄₄N4O₃Cl [(M+H)⁺] 579.3097.

### Example 35

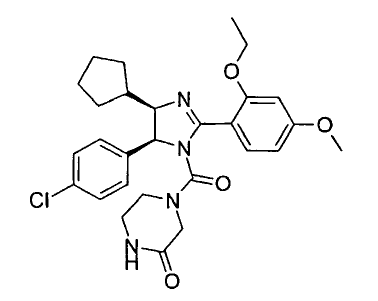

4-[5-(4-Chloro-phenyl)-4-cyclopentyl-2-(2-ethoxy-4-methoxy-phenyl)-4,5-dihydro-imidazole-1-carbonyl]-piperazin-2-one was prepared from 5-(4-chloro-phenyl)-4-cyclopentyl-2-(2-ethoxy-4-methoxy-phenyl)-4,5-dihydro-1H-imidazole, trifluoroacetate salt (Example 19) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 525.2271, calculated for C₂₈H₃₄N₄O₄Cl [(M+H)⁺] 525.2263.

### Example 36

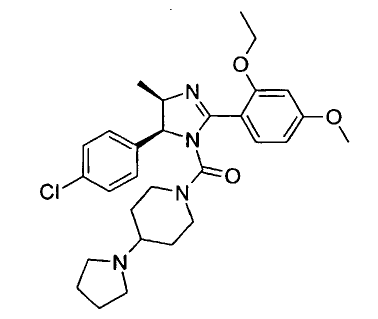

[5-(4-Chloro-phenyl)-2-(2-ethoxy-4-methoxy-phenyl)-4-methyl-4,5-dihydro-imidazol-1-yl]-(4-pyrrolidin-1-yl-piperidin-1-yl)-methanone was prepared from 5-(4-chloro-phenyl)-2-(2-ethoxy-4-methoxy-phenyl)-4-methyl-4,5-dihydro-1H-imidazole, trifluoroacetate salt (Example 17) in an analogous manner as described for the preparation of [5-(4-chloro-phenyl)-4-cyclopentylmethyl-2-(2-ethoxy-4-methoxyphenyl)-4,5-dihydro-in-imdazol-1-yl]-(4-methyl-piperazin-1-yl)-methanone (Example 24). HR-MS (ES, *m*/*z*) observed 525.2635, calculated for C₂₉H3₈N₄O₃Cl [(M+H)⁺] 525.2627.

### Example 37

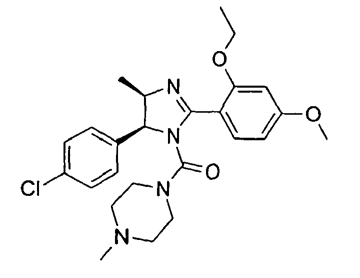

[5-(4-Chloro-phenyl)-2-(2-ethoxy-4-methoxy-phenyl)-4-methyl-4,5-dihydro-imidazol-1-yl]-(4-methyl-piperazin-1-yl)-methanone was prepared from 5-(4-chlorophenyl)-2-(2-ethoxy-4-methoxy-phenyl)-4-methyl-4,5-dihydro-1H-imidazole, trifluoroacetate salt (Example 17) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 471.2166, calculated for C₂₅H₃₂N₄O₃Cl [(M+H)⁺] 471.2158.

### Example 38

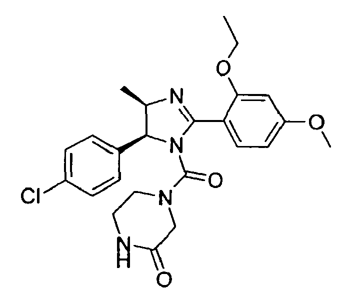

4-[5-(4-Chloro-phenyl)-2-(2-ethoxy-4-methoxy-phenyl)-4-methyl-4,5-dihydroimidazole-1-carbonyl]-piperazin-2-one was prepared from 5-(4-chloro-phenyl)-2-(2-ethoxy-4-methoxy-phenyl)-4-methyl-4,5-dihydro-1H-infidazole, trifluoroacetate salt (Example 17) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 471.1801, calculated for C₂₄H₂₈N₄O₄Cl [(M+H)⁺] 471.1794.

### Example 39

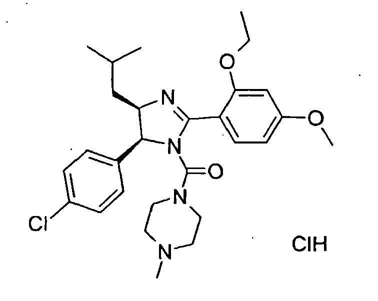

[5-(4-Chloro-phenyl)-2-(2-ethoxy-4-methoxy-phenyl)-4-isobutyl-4,5-dihydro-imidazol-1-yl]-(4-methyl-piperazin-1-yl)-methanone was prepared from 5-(4-chlorophenyl)-2-(2-ethoxy-4-methoxy-phenyl)-4-isobutyl-4,5-dihydro-1H-imidazole (Example 10) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 513.2632, calculated for C₂₈H₃₈N₄O₃Cl [(M+H)⁺] 513.2627.

### Example 40

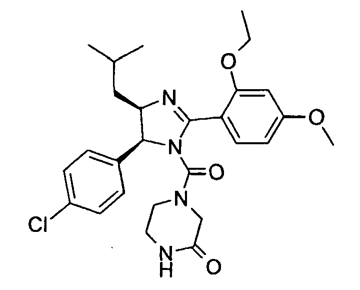

4-[5-(4-Chloro-phenyl)-2-(2-ethoxy-4-methoxy-phenyl)-4-isobutyl-4,5-dihydroimidazole-1-carbonyl]-piperazin-2-one was prepared from 5-(4-chloro-phenyl)-2-(2-ethoxy-4-methoxy-phenyl)-4-isobutyl-4,5-dihydro-1H-imidazole (Example 10) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 513.2267, calculated for C₂₇H₃₃N₄O₃Cl [(M+H)⁺] 513.2263.

### Example 41

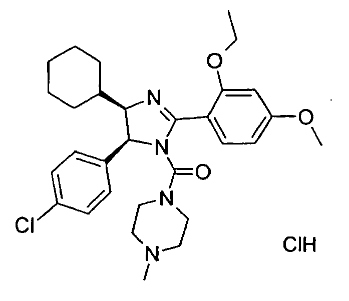

[5-(4-Chloro-phenyl)-2-(2-ethoxy-4-methoxy-phenyl)-4-isobutyl-4,5-dihydro-imidazol-1-yl]-(4-pyrrolidin-1-yl-piperidin-1-yl)-methanone was prepared from 5-(4-chloro-phenyl)-2-(2-ethoxy-4-methoxy-phenyl)-4-isobutyl-4,5-dihydro-1H-imidazole (Example 10) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 567.3103, calculated for C₃₂H₄₃N₄O₃Cl [(M+H)⁺] 567.3097.

### Example 42

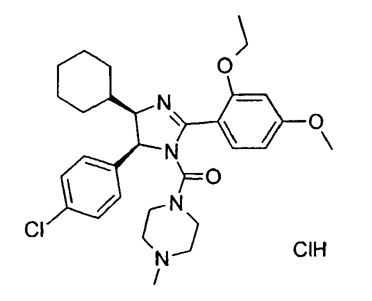

[5-(4-Chloro-phenyl)-4-cyclohexyl-2-(2-ethoxy-4-methoxy-phenyl)-4,5-dihydro-imidazol-1-yl]-(4-methyl-piperazin-1-yl)-methanone was prepared from 5-(4-chlorophenyl)-4-cyclohexyl-2-(2-ethoxy-4-methoxy-phenyl)-4,5-dihydro-1H-imidazole (Example 11) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 539.2790, calculated for C₃₀H₄₀N₄O₃Cl [(M+H)⁺] 539.2784.

### Example 43

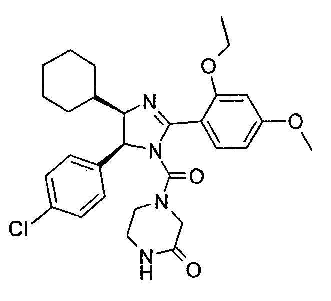

4-[5-(4-Chloro-phenyl)-4-cyclohexyl-2-(2-ethoxy-4-methoxy-phenyl)-4,5-dihydroimidazole-1-carbonyl]-piperazin-2-one was prepared from 5-(4-chloro-phenyl)-4-cyclohexyl-2-(2-ethoxy-4-methoxy-phenyl)-4,5-dihydro-1H-imidazole (Example 11) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 539.2426, calculated for C₂₉H₃₆N₄O₄Cl [(M+H)⁺] 539.2420.

### Example 44

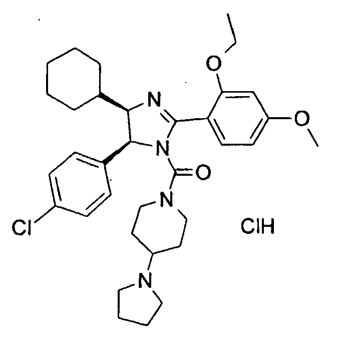

[5-(4-Chloro-phenyl)-4-cyclohexyl-2-(2-ethoxy-4-methoxy-phenyl)-4,5-dihydro-imidazol-1-yl]-(4-pyrrolidin-1-yl-piperidin-1-yl)-methanone was prepared from 5-(4-chloro-phenyl)-4-cycl,ohexyl-2-(2-ethoxy-4-methoxy-phenyl)-4,5-dihydro-1H-imidazole (Example 11) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 593.3260, calculated for C₃₆H₄₆N₄O₃Cl [(M+H)⁺] 593.3253.

### Example 45

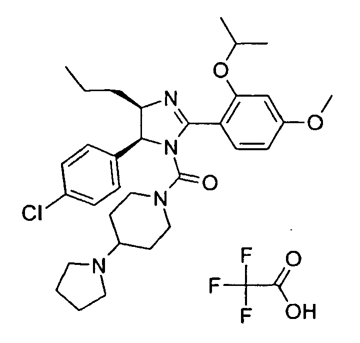

[5-(4-Chloro-phenyl)-2-(2-isopropoxy-4-methoxy-phenyl)-4-propyl-4,5-dihydro-imidazol-1-yl]-(4-pyrrolidin-1-yl-piperidin-1-yl)-methanone, trifluoroacetate salt was prepared from 5-(4-chloro-phenyl)-2-(2-isopropoxy-4-methoxy-phenyl)-4-propyl-4,5-dihydro-1H-imidazole (Example 23) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 567.3109, calculated for C₃₂H₄₄N₄O₃Cl [(M+H)⁺] 567.3097.

### Example 46

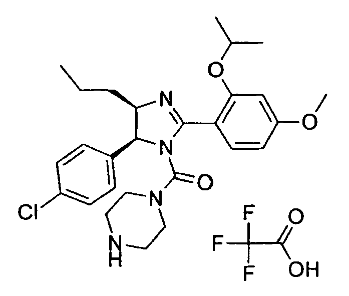

[5-(4-Chloro-phenyl)-2-(2-isopropoxy-4-methoxy-phenyl)-4-propyl-4,5-dihydro-imidazol-1-yl]-piperazin-1-yl-methanone, trifluoroacetate salt was prepared from 5-(4-chloro-phenyl)-2-(2-isopropoxy-4-methoxy-phenyl)-4-propyl-4,5-dihydro-1H-imidazole (Example 23) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 499.2475, calculated for C₂₇H₃₆N₄O₃Cl [(M+H)⁺] 499.2471.

### Example 47

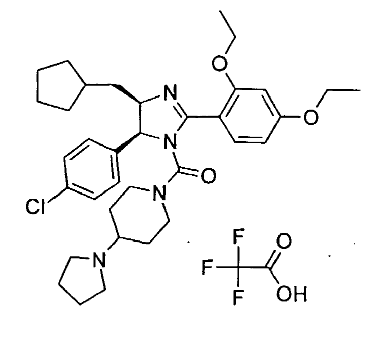

[5-(4-Chloro-phenyl)-4-cyclopentylmethyl-2-(2,4-diethoxy-phenyl)-4,5-dihydro-imidazol-1-yl]-(4-pyrrolidin-1-yl-piperidin-1-yl)-methanone, trifluoroacetate salt was prepared from 5-(4-chloro-phenyl)-4-cyclopentylmethyl-2-(2,4-diethoxy-phenyl)-4,5-dihydro-1H-imidazole (Example 21) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 607.3423, calculated for C₃₅H₄₈N₄O₃Cl [(M+H)⁺] 607.3410.

### Example 48

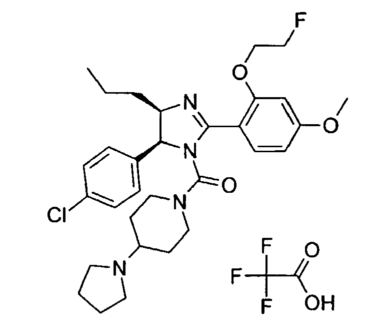

{ 5-(4-Chloro-phenyl)-2-[2-(2-fluoro-ethoxy)-4-methoxy-phenyl]-4-propyl-4,5-dihydro-imidazol-1-yl}-(4-pyrrolidin-1-yl-piperidin-1-yl)-methanone, trifluoroacetate salt was prepared from 5-(4-chloro-phenyl)-2-[2-(2-fluoro-ethoxy)-4-methoxy-phenyl]-4-propyl-4,5-dihydro-1H-imidazole (Example 22) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 471.2854, calculated for C₃₁H₄₁N₄O₃Cl [(M+H)⁺] 471.2846.

### Example 49

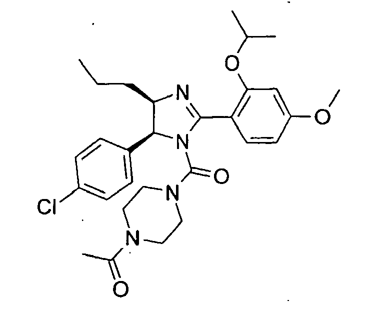

1-{4-[5-(4-Chloro-phenyl)-2-(2-isopropoxy-4-methoxy-phenyl)-4-propyl-4,5-dihydro-imidazole-1-carbonyl]-piperazin-1-yl}-ethanone was prepared from 5-(4-chlorophenyl)-2-(2-isopropoxy-4-methoxy-phenyl)-4-propyl-4,5-dihydro-1H-imidazole (Example 23) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 541.2581, calculated for C₂₉H₃₈N₄O₄Cl [(M+H)⁺] 541.2576.

### Example 50

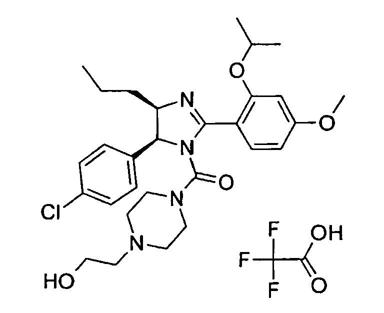

[5-(4-Chloro-phenyl)-2-(2-isopropoxy-4-methoxy-phenyl)-4-propyl-4,5-dihydro-imidazol-1-yl]-[4-(2-hydroxy-ethyl)-piperazin-1-yl]-methanone, trifluoroacetate salt was prepared from 5-(4-chloro-phenyl)-2-(2-isopropoxy-4-methoxy-phenyl)-4-propyl-4,5-dihydro-1H-imidazole (Example 23) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 543.2738, calculated for C₂₉H₃₉N₄O₄Cl [(M+H)⁺] 543.2733.

### Example 51

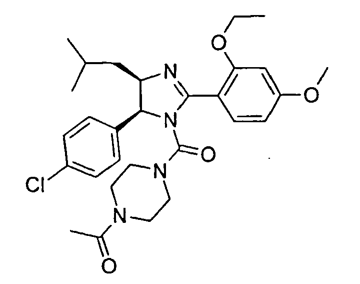

1-{4-[5-(4-Chloro-phenyl)-2-(2-ethoxy-4-methoxy-phenyl)-4-isobutyl-4,5-dihydro-imidazole-1-carbonyl]-piperazin-1-yl}-ethanone was prepared from 5-(4-chloro-phenyl)-2-(2-ethoxy-4-methoxy-phenyl)-4-isobutyl-4,5-dihydro-1H-imidazole (Example 10) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 541.2580, calculated for C₂₆H₃₂N₄O₄Cl [(M+H)⁺] 541.2576.

### Example 52

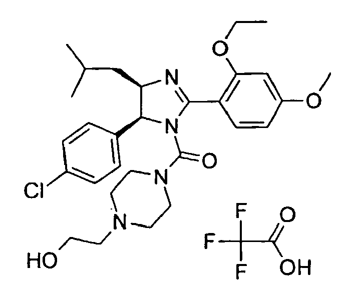

[5-(4-Chloro-phenyl)-2-(2-ethoxy-4-methoxy-phenyl)-4-isobutyl-4,5-dihydro-imidazol-1-yl]-[4-(2-hydroxy-ethyl)-piperazin-1-yl]-methanone, trifluoroacetate salt was prepared from 5-(4-chloro-phenyl)-2-(2-ethoxy-4-methoxy-phenyl)-4-isobutyl-4,5-dihydro-1H-imidazole (Example 10) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperein-1-yl)-methanone (Example 24). HR-MS (ES, *m*/*z*) observed 543.2737, calculated for C₂₉H₄₀N₄O₄Cl [(M+H)⁺] 543.2733.

### Example 53

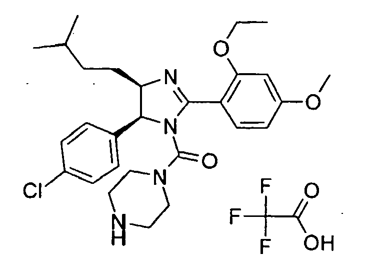

[5-(4-Chloro-phenyl)-2-(2-ethoxy-4-methoxy-phenyl)-4-(3-methyl-butyl)-4,5-dihydro-imidazol-1-yl]-piperazin-1-yl-methanone, trifluoroacetate salt was prepared from 5-(4-chloro-phenyl)-2-(2-ethoxy-4-methoxy-phenyl)-4-(3-methyl-butyl)-4,5-dihydro-1H-imidazole (Example 15) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 513.2632, calculated for C₂₈H₃₇N₄O₃Cl [(M+H)⁺] 513.2637.

### Example 54

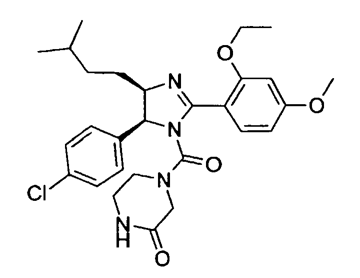

4-[5-(4-Chloro-phenyl)-2-(2-ethoxy-4-methoxy-phenyl)-4-(3-methyl-butyl)-4,5-dihydro-imidazole-1-carbonyl]-piperazin-2-one was prepared from 5-(4-chloro-phenyl)-2-(2-ethoxy-4-methoxy-phenyl)-4-(3-methyl-butyl)-4,5-dihydro-1H-imidazole (Example 15) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 527.2423, calculated for C₂₈H₃₆N₄O₄Cl [(M+H)⁺] 527.2120.

### Example 55

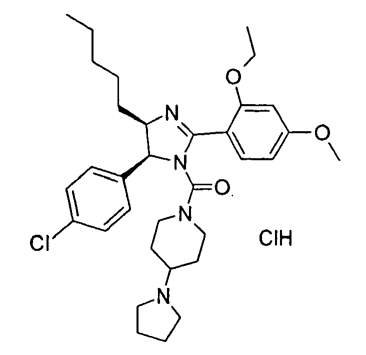

[5-(4-Chloro-phenyl)-2-(2-ethoxy-4-methoxy-phenyl)-4-pentyl-4,5-dihydro-imidazol-1-yl]-(4-pyrrolidin-1-yl-piperidin-1-yl)-methanone was prepared from 5-(4-chloro-phenyl)-2-(2-ethoxy-4-methoxy-phenyl)-4-pentyl-4,5-dihydro-1H-imidazole (Example 12) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 581.3262, calculated for C₃₃H₄₆N₄O₃Cl [(M+H)⁺] 581.3253.

### Example 56

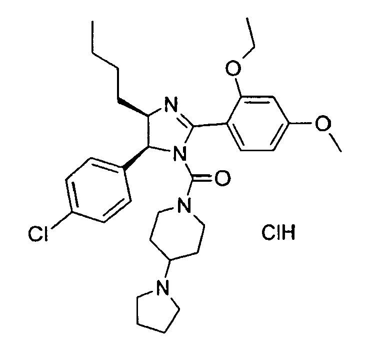

[4-Butyl-5-(4-chloro-phenyl)-2-(2-ethoxy-4-methoxy-phenyl)-4,5-dihydro-imidazol-1-yl]-(4-pyrrolidin-1-yl-piperidin-1-yl)-methanone was prepared from 4-butyl-5-(4-chloro-phenyl)-2-(2-ethoxy-4-methoxy-phenyl)-4,5-dihydro-1H-imidazole (Example 13) in an analogous manner as described for the preparation of [5-(4-chlorophenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 567.3106, calculated for C₃₂H₄₄N₄O₃Cl [(M+H)⁺] 567.3097.

### Example 57

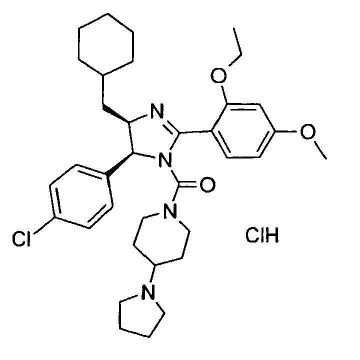

[5-(4-Chloro-phenyl)-4-cyclohexylmethyl-2-(2-ethoxy-4-methoxy-phenyl)-4,5-dihydro-imidazol-1-yl]-(4-pyrrolidin-1-yl-piperidin-1-yl)-methanone was prepared from 5-(4-chloro-phenyl)-4-cyclohexylmethyl-2-(2-ethoxy-4-methoxy-phenyl)-4,5-dihydro-1H-imidazole (Example 14) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 607.3419, calculated for C₃₅H₄₈N₄O₃Cl [(M+H)⁺] 607.3410.

### Example 58

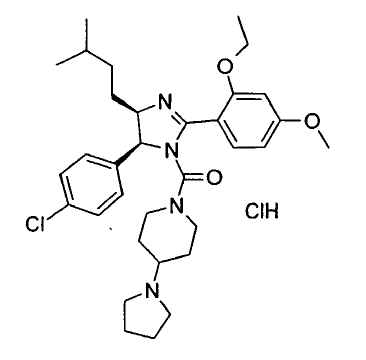

[5-(4-Chloro-phenyl)-2-(2-ethoxy-4-methoxy-phenyl)-4-(3-methyl-butyl)-4,5-dihydro-imidazol-1-yl]-(4-pyrrolidin-1-yl-piperidin-1-yl)-methanone was prepared from 5-(4-chloro-phenyl)-2-(2-ethoxy-4-methoxy-phenyl)-4-(3-methyl-butyl)-4,5-dihydro-1H-imidazole (Example 15) in an analogous manner as described for the preparation of [5-(4-chloro-phenyl)-4-cyclopentylmethyl-2-(2-ethoxy-4-methoxy-phenyl)-4,5-dihydro-imidazol-1-yl]-(4-methyl-piperazin-1-yl)-methanone (Example 24). HR-MS (ES, *m*/*z*) observed 581.3261, calculated for C₃₃H₄₆N₄O₃Cl [(M+H)⁺] 581.3253.

### Example 59

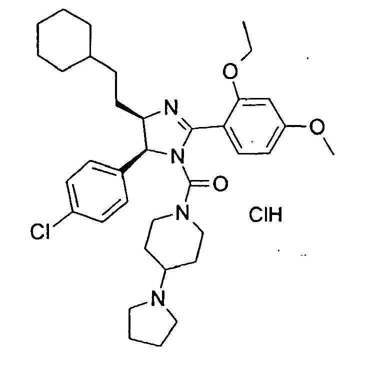

[5-(4-Chloro-phenyl)-4-(2-cyclohexyl-ethyl)-2-(2-ethoxy-4-methoxy-phenyl)-4,5-dihydro-imidazol-1-yl]-(4-pyrrolidin-1-yl-piperidin-1-yl)-methanone was prepared from 5-(4-chloro-phenyl)-4-(2-cyclohexyl-ethyl)-2-(2-ethoxy-4-methoxy-phenyl)-4,5-dihydro-1H-imidazole (Example 16) in an analogous manner as described for the preparation of [5-(4-chloro-phenyl)-4-cyclopentylmethyl-2-(2-ethoxy-4-methoxyphenyl)-4,5-dihydro-imidazol-1-yl]-(4-methyl-piperazin-1-yl)-methanone (Example 24). HR-MS (ES, *m*/*z*) observed 621.3573, calculated for C₃₆H₅₀N₄O₃Cl [(M+H)⁺] 621.3566.

### Example 60

### In Vitro Activity Assay

The ability of the compounds to inhibit the interaction between p53 and MDM2 proteins was measured by an ELISA (Enzyme-Linked Immuno Sorbent Assay) in which recombinant GST-tagged MDM2 binds to a peptide that resembles the MDM2-interacting region of p53 (Böttger et al., J. Mol. Bio. 1997, Vol. 269, pgs. 744-756). This peptide was immobilized to the surface of a 96 well plate via N-terminal biotin which binds to streptavidin-coated wells. MDM2 was added to each well in the presence of anti-MDM2 mouse monoclonal antibody (SMP-14, Santa Cruz Biotech). After removal of the unbound MDM2 protein, a peroxydase-linked secondary antibody (anti-mouse IgG, Roche Molecular Biochemicals) and the amount of peptide-bound MDM2 was determined colorimetrically by the addition of a peroxydase substrate (MTB Microwell Peroxydase Substrate System, Kirkegaard & Perry Labs).

Test plates were prepared by coating with streptavidin (5 mg/ml in PBS) for 2 hours followed by a PBS (phosphate-buffered saline) wash and overnight blocking with 150 µl of blocking buffer containing 2 mg/ml bovine serum albumin (Sigma) and 0.05% Tween 20 (Sigma) in PBS at 4°C. Biotinylated peptide (1 µM) was added to each well in 50 µl of blocking buffer and washed extensively after 1 h incubation. Test compounds were diluted in a separate 96 well plate and added in triplicate to a compound incubation plate containing a mix of the MDM2 protein and anti-MDM2 antibody. After 20 min incubation, the content of the plate was transferred to the test plate and incubated for an additional 1 hour. The secondary anti-mouse IgG antibody was added to the test plate proceeded and followed by a triple wash with 0.05% Tween 20 in PBS. Finally, peroxydase substrate was added to each well and the absorption was read using a plate reader (MR7000, Dynatech) at 450nm. The inhibitory activity of the test compounds was measured as a percentage of the bound MDM2 in treated vs. untreated wells and IC₅₀ was calculated.

IC₅₀S showing biological activity that applies to compounds of the subject matter of this invention ranges from about 0.5 µM to about 150 µM. Specific data for some examples are as follows:

| **Example** | **IC50 (µM)** |
|---|---|
| 26 | 45.0 |
| 24 | 13.2 |
| 28 | 5.5 |
| 55 | 0.7 |

## Claims

1. At least one compound selected from a compound of formula I 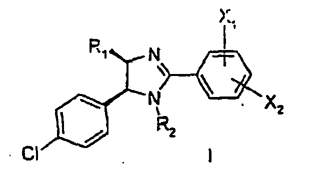 wherein
X₁ and X₂ are each independently selected from the group consisting of hydrogen, -OR₃, -SR₄ -NR₅R₆, -CONR₅R₆, -COOR₇, halogen, nitro, trifluoromethyl, C₁-C₆ alkyl, C₁-C₆ alkyl substituted by R₈, C₃-C₈ cycloalkyl;
R₁ is selected from the group consisting of C1-C8 alkyl, C1-C4 alkyl attached to C4-C8 cycloalkyl and C4-C8 cycloalkyl;
R₂ is H or -C=OR₉;
R₃ is selected from the group consisting of hydrogen, C₁-C₆ alkyl, C₁-C₆ alkyl substituted by R₈, and C₃-C₈ cycloalkyl;
R₄ is selected from the group consisting of hydrogen and C₁-C₆ alkyl;
R₅ and R₆ are each independently selected from the group consisting of hydrogen, C₁-C₆ alkyl and C₃-C₈ cycloalkyl; or
R₅ and R₆ together with the two carbon atoms and the bonds between them from the benzene ring to which they are substituted form a ring selected from a 5- or 6-membered unsaturated ring or a 5- or 6-membered saturated ring that contains at least one hetero
atom selected from S, N and O;
R₇ is selected from the group consisting of hydrogen, C₁-C₆ alkyl and C₃-C₈ cycloalkyl;
R₈ is selected from the group consisting of -CONR₅R₆, -NR₅R₆, COOR₇, aryl, halogen, , C₁-C₆ alkoxy, morpholinyl and heterocycles;
R₉ is selected from C1-C4 alkyl, -CH=CHCOOH, -NHCH₂CH₂R₁₀, - N(CH₂CH₂OH)CH₂CH₂OH, -N(CH₃)CH₂CH₂NCH₃, -N(CH₃)CH₂CH₂N(CH₃)CH₃, saturated 4-, 5- and 6-membered rings, and saturated and unsaturated 5- and 6- membered rings containing at least one hetero atom wherein the hetero atom is selected from S, N and O and being optionally substituted with a group selected from C₁-C₆ alkyl, -C=O-R₁₁, -OH, C₁-C₆ alkyl substituted with hydroxy, C₁-C₆ alkyl, substituted with -NH₂, N-C₁-C₆ alkyl, -SO₂CH₃, =O, -CH₂C=OCH₃, and 5- and 6-membered'saturated rings containing at least one hetero atom selected from S, N and O.
R₁₀ is selected from the group consisting of -N(CH₃)CH₃, -NHCH₂CH₂NH₂, -NH₂, morpholinyl and piperazinyl; and
R₁₁ is selected from H, C₁-C₆ alkyl, -NH_{2,} -N-C₁-C₆ alkyl, C₁-C₆ alkyl substituted with hydroxy, and C₁-C₆ alkyl substituted with NH₂, whereby each cycloalkyl represents a non-aromatic, partially or completely saturated cyclic aliphatic hydrocarbon group;
or a pharmaceutically acceptable salt or ester thereof.

2. The compound of claim 1 wherein R1 is C₁-C₈ alkyl.

3. The compound of claim 1 wherein R1 is C₁-C₄ alkyl attached to C₄-C₈ cycloalkyl.

4. The compound of claim 1 wherein R1 is C₄-C₈ cycloalkyl.

5. The compound of claim 1 wherein X₁ is selected from the group consisting of ethoxy, isopropoxy, 2-fluoroethoxy and -OCH₂CF₃ at the ortho position.

6. The compound of claim 1 wherein X₂ is selected from the group consisting of methoxy, ethoxy and trifluoromethyl at the para position.

7. The compound of claim 1 wherein R₉ is selected from the group consisting of morpholinyl, piperazinyl, piperadinyl, cyclopentyl, cyclohexyl, thiophenyl, isoxazlyl, furanyl, piperazinyl substituted with at least one group selected from C1-C3 alkyl, -C1-C2 alkoxy, -C=OCH₃, -SO₂CH₃, -C=O, -OH, -CH₂NH₂, -C=OCH₂NH₂, -C=OCH₂OH, -C=OC(OH)CH₂OH, -CH₂C(OH)-CH₂OH, -C=ON(CH₂-)₂, -C=ONH₂, -C=ON(CH₃)CH₃, -C=OCH(CH₃)₂, -CH₂C=OCH₃, -CH₂CH(OH)CH₃, -CH(CH₃)CH(OH)CH₃ and piperidinyl substituted with at least one group selected from C1-C3 alkyl, -C1-C2 alkoxy, -C=OCH₃, -SO₂CH₃, -C=O, -OH, -CH₂KH₂, -C=OCH₂NH₂, -C=OCH₂OH, -C=OC(OH)CH₂OH -CH₂C(OH) CH₂OH, -C=ON(CH₂)₂, -C=ONH₂, and -C=ON(CH₃)CH₃, -N(CH₃)CH₃, pyrrolidinyl and piperadinyl.

8. The compound of claim 1 that is selected from the group consisting of:
5-(4-chloro-phenyl)-4-cyclopentylmethyl-2-(2-ethoxy-4-methoxy-phenyl)-4,5-dihydro-1H-inudazole;
5-(4-Chloro-phenyl)-2-(2-ethoxy-4-methoxy-phenyl)-4-isobutyl-4,5-dihydro-1H-imidazole;
5-(4-Chloro-phenyl)-4-cyclohexyl-2-(2-ethoxy-4-methoxy-phenyl)-4,5-dihydro-1H-imidazole;
5-(4-Chloro-phenyl)-2-(2-ethoxy-4-methoxy-phenyl)-4-pentyl-4,5-dihydro-1H-imidazole
4-Butyl-5-(4-chloro-phenyl)-2-(2-etlloxy-4-methoxy-phenyl)-4,5-dihydro-1H-imidazole;
5-(4-Chloro-phenyl)-4-cyclohexylmethyl-2-(2-ethoxy-4-methoxy-phenyl)-4,5-dihydro-1H-imidazole;
5-(4-Chloro-phenyl)-2-(2-ethoxy-4-methoxy-phenyl)-4-(3-methyl-butyl)-4,5-dihydro-1H-imidazole;
5-(4-Chloro-phenyl)-4-(2-cyclohexyl-ethyl)-2-(2-ethoxy-4-methoxy-phenyl)-4,5-dihydro- H-imidazole;
5-(4-Chloro-phenyl)2-(2-ethoxy-4-methoxy-phenyl)-4-methyl-4,5-dihydro-1H-imidazole, trifluoroacetate salt; and
5-(4-Chloro-phenyl)-2-(2-ethoxy-4-methoxy-phenyl)-4-propyl-4,5-dihydro-1H-imidazole.

9. The compound of claim 1 that is selected from the group consisting of:
5-(4-Chloro-phenyl)-4-cyclopentyl-2-(2-ethoxy-4-methoxy-phenyl)-4,5-dihydro-IH-imidazole, trifluoroacetate salt;
5-(4-Chloro-phenyl)-2-(2-ethoxy-4-methoxy-phenyl)-4-ethyl-4,5-dihydro-1H-imidazole
5-(4-Chloro-phenyl)-4-cycopentylmethyl-2-(2,4-diethoxy-phenyl)-4,5-dihydro-1H-imidazole;
5-(4-Chloro-phenyl)-2-[2-(2-fluoro-ethoxy)-4-methoxy-phenyl]-4-propyl-4,5-dihydro-1H-imidazole
5-(4-Choro-phenyl)-2-(2-isopropoxy-4-methoxy-phenyl)-4-propyl-4,5-dihydro-1H-imidazole;
[5-(4-Chloro-phenyl)-4-cydopentylmethyl-2-(2-ethoxy-4-methoxy-phenyl)-4,5-djhydro-imidazol-1-yl]-(4-methyl-piperazin-1-yl)-methanone, trifluoroacetate salt;
4-[5-(4-Chloro-phenyl)-2-(2-ethoxy-4-methoxy-phenyl)-4-propyl.4,5-dihydro-imidazole-1-carbonyl]-piperazin-2-one;
[5-(4-Chloro-phenyl)-2-(2-ethoxy-4-methoxy-phenyl)-4-propyl-4,5-dihydro-imidazol-1-yl]-(4-pyrrolidin-1-yl-piperidin-1-yl)-methanone, trifluoroacetate salt;
[5-(4-Chloro-phenyl)-2-(2-ethoxy-4-methoxy-phenyl)-4-propyl-4,5-dihydro-imidazol-1-yl]-(4-methyl-piperazin-1-yl)-methanone
[5-(4-Chloro-phenyl)-4-cyclopentylmethyl-2-(2-ethoxy-4-methoxy-phenyl)-4,5-dihydro-imidazol-1-yl]-(4-pyrrolidin-l-yl-piperidin-1-yl)-methanone, trifluoroacetate salt.

10. The compound of claim 1 that is selected from the group consisting of:
4-[5-(4-Chloro-phenyl)-4-cyclopentylmethyl-2-(2-ethoxy-4-methoxyphenyl)-4,5-dihydro-imidazole-1-carbonyl]-piperazin-2-one;
[5-(4-Chloro-phenyl)-2-(2-ethoxy-4-methoxy-phenyl)-4-ethyl-4,5-dihydro-imidazol-1-yl]-(4-methyl-piperazin-1-yl)-methanone;
[5-(4-Chloro-phenyl)-2-(2-ethoxy-4-methoxy-phenyl)-4-ethyl-4,5-dihydro-imidazol-1-yl]-(4-pyrrolidin-1-yl-piperidin-1-yl)-methanone;
4-[5-(4-Chloro-phenyl)-2-(2-ethoxy-4-methoxy-phenyl)-4-ethyl-4,5-dihydroimidazole-1-carbonyl]-piperazin-2-one;
[5-(4-Chloro-phenyl)-4-cyclopentyl-2-(2-ethoxy-4-methoxy-phenyl)-4,5-dihydro-imidazol-1-yl]-(4-methyl-piperazin-1-yl)-methanone;
[5-(4-Choro-phenyl)-4-cyclopentyl-2-(2-ethoxy-4-methoxy-phenyl)-4,5-dihydro-imidazol-1-yi]-(4-pyrrolidin-1-yl-piperidin-1-yl)-methanone;
4-[5-(4-Chloro-phenyl)-4-cyclopentyl-2-(2-ethoxy-4-methoxy-phenyl)-4,5-dihydro-imidazole-1-carbonyl]-piperazin-2-one;
[5-(4-Chloro-phenyl)-2-(2-ethoxy-4-methoxy-phenyl)-4-methyl-4,5-dihydro-imidazol-1-yl]-(4-pyrrolidin-1-yl-piperidin-1-yl)-methanone;
[5-(4-Chloro-phenyl)-2-(2-ethoxy-4-methoxy-phenyl)-4-methyl-4,5-dihydro-imidazol-1-yl]-(4-methyl-piperazin-1-yl)-methanone; and
4-[5-(4-Chloro-phenyl)-2-(2-ethoxy-4-methoxy-phenyl)-4-methl-4,5-dihydro-imidazole-1-carbonyl]-piperazin-2-one.

11. The compound of claim 1 that is selected from the group consisting of:
[5-(4-Chloro-phenyl)-2-(2-ethoxy-4-methoxy-phenyl)-4-isobutyl-4,5-dihydro-imidazol-1-yl]-(4-methyl-piperazin-l-yl)-methanone;
4-[5-(4-Chloro-phenyl)-2-(2-ethoxty-4-methoxy-phenyl)-4-isobutyl-4,5-dihydro-imidazole-1-carbonyl]-piperazin-2-one;
[5-(4-Chloro-phenyl)-2-(2-ethoxy-4-methoxy-phenyl)-4-isobutyl-4,5-dihydro-imidazol-1-yl]-(4-pyrrolidin-1-yl-piperidin-1-yl)-methanone;
[5-(4-Chloro-phenyl)-4-cydohexyl-2-(2-ethoxy-4-methoxy-phenyl)-4,5-dihydro-imidazol-1-yl]-(4-methyl-piperazin-1-yl)-mewanone;
4-[5-(4-Chloro-phenyl)-4-cyclohexyl-2-(2-ethoxy-4-methoxy-phenyl)-4,5-dihydro-imidazole-1-carbonyl]-piperazin-2-one;
[5-(4-Chloro-phenyl)-4-cyclohexyl-2-(2-ethoxy-4-methoxy-phenyl)-4,5-dihydro-imidazol-1-yl]-(4-pyrrolidin-1-yl-piperidin-1-yl)-methanone;
[5-(4-Chloro-phenyl)-2-(2-isopropoxy-4-methoxy-phenyl)-4-propyl-4,5-dihydro-imidazol-1-yl]-(4-pyrrolidin-1-yl-piperidin-1-yl)-methanone, trifluoroacetate salt;
[5-(4-Chloro-phenyl)-2-(2-isopropoxy-4-methoxy-phenyl)-4-propyl-4,5-dihydro-imidazol-1-yl]-piperazin-1-yl-methanone, trifluoroacetate salt;
[5-(4-Chloro-phenyl)-4-cyclopentylmethyl-2-(2,4-diethoxy-phenyl)-4,5-dihydro-imidazol-1-yl]-(4-pyrrolidin-1-yl-piperidin-1-yl)-methanone, trifluoroacetate salt; and
{5-(4-Chloro-phenyl)-2-[2-(2-fluoro-ethoxy)-4-methoxy-phenyl]-4-propyl-4,5-dihydro-imidazol-l-yl}-(4-pyrrolidin-1-yl-piperidin-1-yl)-methanone, trifluoroacetate salt.

12. The compound of claim 1 that is selected from the group consisting of:
1-{4-[5-(4-Chloro-phenyl)-2-(2-isopropoxy-4-methoxy-phenyl)-4-propyl-4,5-dihydro-imidazole-1-carbonyl]-piperazin-1-yl}-ethanone;
[5-(4-Chloro-phenyl)-2-(2-isopropoxy-4-methoxy-phenyl)-4-propyl-4,5-dihydro-imidazol-1-yl]-[4-(2-hydroxy-ethyl)-piperazin-1-yl]-methanone, trifluoroacetate salt;
1-{4-[5-(4-Chloro-phenyl)-2-(2-ethoxy-4-methoxylphenyl)-4-isobutyl-4,5-dihydro-imidazole-1-carbonyl]-piperazin-1-yl}-ethanone;
[5-(4-Chloro-phenyl)-2-(2-ethoxy-4-methoxy-phenyl)-4-isobutyl-4,5-dihydro-imidazol-1-yl]-[4-(2-hydroxy-ethyl)-piperazin-1-yl]-methanone, trifluoroacetate salt;
[5-(4-Chloro-phenyl)-2-(2-ethoxy-4-methoxy-phenyl)-4-(3-methyl-butyl)-4,5-dihydro-imidazol-1-yl]-piperazin-1-yl-methanone, trifluoroacetate salt;
4-[5-(4-Chloro-phenyl)-2-(2-ethoxy-4-methoxy-phenyl)-4-(3-methyl-butyl)-4,5-dihydro-imidazole-1-carbonyl]-piperazin-2-one;
[5-(4-Chloro-phenyl)-2-(2-ethoxy-4-methoxy-phehyl)-4-pentyl-4,5-dihydro-imidazol-1-yl]-(4-pyrrolidin-1-yl-piperidin-1-yl)-methanone;
[4-Butyl-5-(4-chloro-phenyl)-2-(2-ethoxy-4₋methoxy-phenyl)-4,5-dihydro-imidazol-1-yl]-(4-pyrrolidin-1-yl-piperidin-1-yl)-methanone;
[5-(4-Chloro-phenyl)-4-cyclohexylmethyl-2-(2-ethoxy-4-methoxy-phenyl)-4,5-dihydro-imidazol-1-yl]-(4-pyrrolidin-1-yl-piperidin-1-yl)-methanone;
[5-(4-Chloro-phenyl)-2-(2-ethoxy-4-methoxy-phenyl)-4-(3-methyl-butyl)-4,5-dihydro-imidazol-1-yl]-(4-pyrrolidin-1-yl-piperidin-1-yl)-methanone; and
[5-(4-Chloro-phenyl)4-(2-cyclohexyl-ethyl)-2-(2-ethoxy-4-methoxy-phenyl)-4,5-dihydro-imidazol-1-yl]-(4-pyrrolidin-1-yl-piperidin-1-yl)-methanone.

13. A pharmaceutical composition which comprises at least one compound selected from a compound of formula I
according to claim 1,
or a pharmaceutically acceptable salt or ester thereof,
and a pharmaceutically acceptable excipient.

14. Compounds according to anyone of claims 1 to 13 as medicament.

15. Use of a compound of formula I according to claim 1 or a pharmaceutically acceptable salt or ester thereof for the preparation of a medicament for the treatment of a disease based on the interaction of MDM2 protein with a p53-like peptide.

16. Use according to claim 15 wherein the disease is a cell proliferative disorder..

## Patentansprüche

1. Mindestens eine Verbindung ausgewählt aus einer Verbindung der Formel I 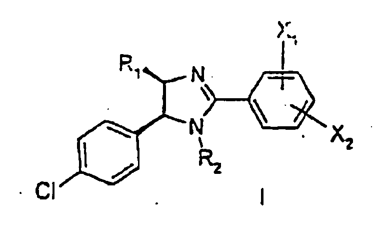 wobei
X₁ und X₂ jeweils unabhängig voneinander ausgewählt sind aus der Gruppe bestehend aus Wasserstoff, -OR₃, -SR₄, -NR₅R₆, -CONR₅R₆, -COOR₇, Halogen, Nitro, Trifluormethyl, C₁-C₆-Alkyl, C₁-C₆-Alkyl substituiert mit R₈, C₃-C₈-Cycloalkyl;
R₁ ausgewählt ist aus der Gruppe bestehend aus C₁-C₈-Alkyl, C₁-C₄-Alkyl gebunden an C₄-C₈-Cycloalkyl und C₄-C₈-Cycloallcyl;
R₂ für H oder -C=OR₉ steht;
R₃ ausgewählt ist aus der Gruppe bestehend aus Wasserstoff, C₁-C₆-Alkyl, C₁-C₆-Alkyl substituiert mit R₈ und C₃-C₈-Cycloalkyl;
R₄ ausgewählt ist aus der Gruppe bestehend aus Wasserstoff und C₁-C₆-Alkyl;
R₅ und R₆ jeweils unabhängig voneinander ausgewählt sind aus der Gruppe bestehend aus Wasserstoff, C₁-C₆-Alkyl und C₃-C₈-Cycloalkyl; oder
R₅ und R₆ zusammen mit den zwei Kohlenstoffatomen und den Bindungen zwischen diesen von dem Benzolring, an welchen sie substituiert sind, einen Ring ausgewählt aus einem 5- oder 6-gliedrigen ungesättigten-Ring oder einem 5- oder 6-gliedrigen gesättigten Ring, welcher mindestens ein Heteroatom ausgewählt aus S, N und O enthält, bilden;
R₇ ausgewählt ist aus der Gruppe bestehend aus Wasserstoff, C₁-C₆-Alkyl und C₃-C₈-Cycloalkyl;
R₈ ausgewählt ist aus der Gruppe bestehend aus -CONR₅R₆, -NR₅R₆, COOR₇, Aryl, Halogen, C₁-C₆-Alkoxy, Morpholinyl und Heterocyclen;
R₉ ausgewählt ist aus C₁-C₄-Alkyl, -CH=CHCOOH, -NHCH₂CH₂R₁₀, -N(CH₂CH₂OH)CH₂CH₂OH, -N(CH₃)CH₂CH₂NCH₃, -N(CH₃)CH₂CH₂N(CH₃)CH₃, gesättigten 4-, 5- und 6-gliedrigen Ringen und gesättigten und ungesättigten 5- und 6-gliedrigen Ringen enthaltend mindestens ein Heteroatom, wobei das Heteroatom ausgewählt ist aus S, N und O und gegebenenfalls mit einer Gruppe ausgewählt aus C₁-C₆-Alkyl, -C=O-R₁₁, -OH, C₁-C₆-Alkyl substituiert mit Hydroxy, C₁-C₆-Alkyl substituiert mit -NH₂, N-C₁-C₆-Alkyl, -SO₂CH₃, =O, -CH₂C=OCH₃, und 5-und 6-gliedrigen gesättigten Ringen enthaltend mindestens ein Heteroatom ausgewählt aus S, N und O substituiert ist;
R₁₀ ausgewählt ist aus der Gruppe bestehend aus -N(CH₃)CH₃, -NHCH₂CH₂NH₂, -NH₂, Morpholinyl und Piperazinyl; und
R₁₁ ausgewählt ist aus H, C₁-C₆-Alkyl, -NH₂, -N-C₁-C₆-Alkyl, C₁-C₆-Alkyl substituiert mit Hydroxy und C₁-C₆-Alkyl substituiert mit NH₂; wobei jedes Cycloalkyl einen nichtaromatischen, teilweise oder vollständig gesättigten cyclischen aliphatischen Kohlenwasserstoffrest darstellt;
oder ein pharmazeutisch verträgliches Salz oder ein pharmazeutisch verträglicher Ester davon.

2. Die Verbindung gemäß Anspruch 1, wobei R₁ für C₁-C₈-Alkyl steht.

3. Die Verbindung gemäß Anspruch 1, wobei R₁ für an C₄-C₈-Cycloalkyl gebundenes C₁-C₄-Alkyl steht.

4. Die Verbindung gemäß Anspruch 1, wobei R₁ für C₄-C₈-Cycloalkyl steht.

5. Die Verbindung gemäß Anspruch 1, wobei X₁ ausgewählt ist aus der Gruppe bestehend aus Ethoxy, Isopropoxy, 2-Fluorethoxy und -OCH₂CF₃ an der ortho-Position.

6. Die Verbindung gemäß Anspruch 1, wobei X₂ ausgewählt ist aus der Gruppe bestehend aus Methoxy, Ethoxy und Trifluormethyl an der para-Position.

7. Die Verbindung gemäß Anspruch 1, wobei R₉ ausgewählt ist aus der Gruppe bestehend aus Morpholinyl, Piperazinyl, Piperadinyl, Cyclopentyl, Cyclohexyl, Thiophenyl, Isoxazolyl, Furanyl, Piperazinyl substituiert mit mindestens einem Rest ausgewählt aus C₁-C₃-Alkyl, -C₁-C₂-Alkoxy, -C=OCH₃, -SO₂CH₃, -C=O, -OH, -CH₂NH₂, -C=OCH₂NH₂, -C=OCH₂OH, -C=OC(OH)CH₂OH, -CH₂C(OH)-CH₂OH, -C=ON(CH₂-)₂, -C=ONH₂, -C=ON(CH₃)CH₃, -C=OCH(CH₃)₂, -CH₂C=OCH₃, -CH₂CH(OH)CH₃, -CH(CH₃)CH(OH)CH₃ und Piperidinyl substituiert mit mindestens einem Rest ausgewählt aus C₁-C₃-Alkyl, -C₁-C₂-Alkoxy, -C=OCH₃, -SO₂CH₃, -C=O, -OH, -CH₂NH₂, -C=OCH₂NH₂, -C=OCH₂OH, -C=OC(OH)CH₂OH, -CH₂C(OH)CH₂OH, -C=ON(CH₂)₂, -C=ONH₂ und -C=ON(CH₃)CH₃, -N(CH₃)CH₃, Pyrrolidinyl und Piperadinyl.

8. Die Verbindung gemäß Anspruch 1, welche ausgewählt ist aus der Gruppe bestehend aus:
5-(4-Chlorphenyl)-4-cyclopentylmethyl-2-(2-ethoxy-4-methoxyphenyl)-4,5-dihydro-1H-imidazol;
5-(4-Chlorphenyl)-2-(2-ethoxy-4-methoxyphenyl)-4-isobutyl-4,5-dihydro-1H-imidazol;
5-(4-Chlorphenyl)-4-cyclohexyl-2-(2-ethoxy-4-methoxyphenyl)-4,5-dihydro-1H-imidazol;
5-(4-Chlorphenyl)-2-(2-ethoxy-4-methoxyphenyl)-4-pentyl-4,5-dihydro-1H-imidazol;
4-Butyl-5-(4-Chlorphenyl)-2-(2-ethoxy-4-methoxyphenyl)-4,5-dihydro-1H-imidazol;
5-(4-Chlorphenyl)-4-cyclohexylmethyl-2-(2-ethoxy-4-methoxyphenyl)-4,5-dihydro-1H-imidazol;
5-(4-Chlorphenyl)-2-(2-ethoxy-4-methoxyphenyl)-4-(3-methylbutyl)-4,5-dihydro-1H-imidazol;
5-(4-Chlorphenyl)-4-(2-cyclohexylethyl)-2-(2-ethoxy-4-methoxyphenyl)-4,5-dihydro-1H-imidazol;
5-(4-Chlorphenyl)-2-(2-ethoxy-4-methoxyphenyl)-4-methyl-4,5-dihydro-1H-imidazol, Trifluoracetatsalz; und
5-(4-Chlorphenyl)-2-(2-ethoxy-4-methoxyphenyl)-4-propyl-4,5-dihydro-1H-imidazol.

9. Die Verbindung gemäß Anspruch 1, welche ausgewählt ist aus der Gruppe bestehend aus:
5-(4-Chlorphenyl)-4-cyclopentyl-2-(2-ethoxy-4-methoxyphenyl)-4,5-dihydro-1H-imidazol, Trifluoracetatsalz;
5-(4-Chlorphenyl)-2-(2-ethoxy-4-methoxyphenyl)-4-ethyl-4,5-dihydro-1H-imidazol;
5-(4-Chlorphenyl)-4-cyclopentylmethyl-2-(2,4-diethoxyphenyl)-4,5-dihydro-1H-imidazol;
5-(4-Chlorphenyl)-2-[2-(2-fluorethoxy)-4-methoxyphenyl]-4-propyl-4,5-dihydro-1H-imidazol;
5-(4-Chlorphenyl)-2-(2-isopropoxy-4-methoxyphenyl)-4-propyl-4,5-dihydro-1H-imidazol;
[5-(4-Chlorphenyl)-4-cyclopentylmethyl-2-(2-ethoxy-4-methoxyphenyl)-4,5-dihydro-imidazol-1-yl]-(4-methylpiperazin-1-yl)methanon, Trifluoracetatsalz;
4-[5-(4-Chlorphenyl)-2-(2-ethoxy-4-methoxyphenyl)-4-propyl-4,5-dihydroimidazol-1-carbonyl]piperazin-2-on;
[5-(4-Chlorphenyl)-2-(2-ethoxy-4-methoxyphenyl)-4-propyl-4,5-dihydro-imidazol-1-yl]-(4-pyrrolidin-1-yl-piperidin-1-yl)methanon, Trifluoracetatsalz;
[5-(4-Chlorphenyl)-2-(2-ethoxy-4-methoxyphenyl)-4-propyl-4,5-dihydro-imidazol-1-yl]-(4-methylpiperazin-1-yl)methanon;
[5-(4-Chlorphenyl)-4-cyclopentylmethyl-2-(2-ethoxy-4-methoxyphenyl)-4,5-dihydro-imidazol-1-yl]-(4-pyrrolidin-1-yl-piperidin-1-yl)methanon, Trifluoracetatsalz.

10. Die Verbindung gemäß Anspruch 1, welche ausgewählt ist aus der Gruppe bestehend aus:
4-[5-(4-Chlorphenyl)-4-cyclopentylmethyl-2-(2-ethoxy-4-methoxyphenyl)-4,5-dihydro-imidazol-1-carbonyl]piperazin-2-on;
[5-(4-Chlorphenyl)-2-(2-ethoxy-4-methoxyphenyl)-4-ethyl-4,5-dihydro-imidazol-1-yl]-(4-methylpiperazin-1-yl)methanon;
[5-(4-Chlorphenyl)-2-(2-ethoxy-4-methoxyphenyl)-4-ethyl-4,5-dihydro-imidazol-1-yl]-(4-pyrrolidin-1-yl-piperidin-1-yl)methanon;
4-[5-(4-Chlorphenyl)-2-(2-ethoxy-4-methoxyphenyl)-4-ethyl-4,5-dihydro-imidazol-1-carbonyl]piperazin-2-on;
[5-(4-Chlorphenyl)-4-cyclopentyl-2-(2-ethoxy-4-methoxyphenyl)-4,5-dihydro-imidazol-1-yl]-(4-methylpiperazin-1-yl)methanon;
[5-(4-Chlorphenyl)-4-cyclopentyl-2-(2-ethoxy-4-methoxyphenyl)-4,5-dihydro-imidazol-1-yl]-(4-pyrrolidin-1-yl-piperidin-1-yl)methanon;
4-[5-(4-Chlorphenyl)-4-cyclopentyl-2-(2-ethoxy-4-methoxyphenyl)-4,5-dihydro-imidazol-1-carbonyl]piperazin-2-on;
[5-(4-Chlorphenyl)-2-(2-ethoxy-4-methoxyphenyl)-4-methyl-4,5-dihydro-imidazol-1-yl]-(4-pyriolidin-1-yl-piperidin-1-yl)methanon;
[5-(4-Chlorphenyl)-2-(2-ethoxy-4-methoxyphenyl)-4-methyl-4,5-dihydro-imidazol-1-yl]-(4-methylpiperazin-1-yl)methanon; und
4-[5-(4-Chlorphenyl)-2-(2-ethoxy-4-methoxyphenyl)-4-methyl-4,5-dihydro-imidazol-1-carbonyl]piperazin-2-on.

11. Die Verbindung gemäß Anspruch 1, welche ausgewählt ist aus der Gruppe bestehend aus:
[5-(4-Chlorphenyl)-2-(2-ethoxy-4-methoxyphenyl)-4-isobutyl-4,5₋dihydro-imidazol-1-yl]-(4-methylpiperazin-1-yl)methanon;
4-[5-(4-Chlorphenyl)-2-(2-ethoxy-4-methoxyphenyl)-4-isobutyl-4,5-dihydro-imidazol-1-carbonyl]piperazin-2-on;
[5-(4-Chlorphenyl)-2-(2-ethoxy-4-methoxyphenyl)-4-isobutyl-4,5-dihydro-imidazol-1-yl]-(4-pyrrolidin-1-yl-piperidin-1-yl)methanon;
[5-(4-Chlorphenyl)-4-cyclohexyl-2-(2-ethoxy-4-methoxyphenyl)-4,5-dihydro-imidazol-1-yl]-(4-methylpiperazin-1-yl)methanon;
4-[5-(4-Chlorphenyl)-4-cyclohexyl-2-(2-ethoxy-4-methoxyphenyl)-4,5-dihydro-imidazol-1-carbonyl]piperazin-2-on;
[5-(4-Chlorphenyl)-4-cyclohexyl-2-(2-ethoxy-4-methoxyphenyl)-4,5-dihydro-imidazol-1-yl]-(4-pyrrolidin-1-yl-piperidin-1-yl)methanon;
[5-(4-Chlorphenyl)-2-(2-isopropoxy-4-methoxyphenyl)-4-propyl-4,5-dihydro-imidazol-1-yl]-(4-pyrrolidin-1-yl-piperidin-1-yl)methanon, Trifluoracetatsalz;
[5-(4-Chlorphenyl)-2-(2-isopropoxy-4-methoxyphenyl)-4-propyl-4,5-dihydro-imidazol-1-yl]piperazin-1-yl-methanon, Trifluoracetatsalz;
[5-(4-Chlorphenyl)-4-cyclopentylmethyl-2-(2,4-diethoxyphenyl)-4,5-dihydro-imidazol-1-yl]-(4-pyrrolidin-1-yl-piperidin-1-yl)methanon, Trifluoracetatsalz; und
{5-(4-Chlorphenyl)-2-[2-(2-fluorethoxy)-4-methoxyphenyl]-4-propyl-4,5-dihydro-imidazol-1-yl}-(4-pyrrolidin-1-yl-piperidin-1-yl)methanon, Trifluoracetatsalz.

12. Die Verbindung gemäß Anspruch 1, welche ausgewählt ist aus der Gruppe bestehend aus:
1-{4-[5-(4-Chlorphenyl)-2-(2-isopropoxy-4-methoxyphenyl)-4-propyl-4,5-dihydro-imidazol-1-carbonyl]piperazin-1-yl} ethanol;
[5-(4-Chlorphenyl)-2-(2-isopropoxy-4-methoxyphenyl)-4-propyl-4,5-dihydro-imidazol-1-yl]-[4-(2-hydroxyethyl)piperazin-1-yl]methanon, Trifluoracetatsalz;
1-{4-[5-(4-Chlorphenyl)-2-(2-ethoxy-4-methoxyphenyl)-4-isobutyl-4,5-dihydro-imidazol-1-carbonyl]piperazin-1-yl} ethanon;
[5-(4-Chlorphenyl)-2-(2-ethoxy-4-methoxyphenyl)-4-isobutyl-4,5-dihydro-imidazol-1-yl]-[4-(2-hydroxyethyl)piperazin-1-yl]methanon, Trifluoracetatsalz;
[5-(4-Chlorphenyl-2-(2-ethoxy-4-methoxyphenyl)-4-(3-methylbutyl)-4,5-dihydro-imidazol-1-yl]piperazin-1-yl-methanon, Trifluoracetatsalz;
4-[5-(4-Chlorphenyl)-2-(2-ethoxy-4-methoxyphenyl)-4-(3-methylbutyl)-4,5-dihydro-imidazol-1-carbonyl]piperazin-2-on;
[5-(4-Chlorphenyl)-2-(2-ethoxy-4-methoxyphenyl)-4-pentyl-4,5-dihydro-imidazol-1-yl]-(4-pyrrolidin-1-yl-piperidin-1-yl)methanon;
[4-Butyl-5-(4-chlorphenyl)-2-(2-ethoxy-4-methoxyphenyl)-4,5-dlhydro-imidazol-1-yl]-(4-pyrrolidin-1-yl-piperidin-1-yl)methanon;
[5-(4-Chlorphenyl)-4-cyclohexylmethyl-2-(2-ethoxy-4-methoxyphenyl)-4,5-dihydro-imidazol-1-yl]-(4-pyrrolidin-1-yl-piperidin-1-yl)methanon;
[5-(4-Chlorphenyl)-2-(2-ethoxy-4-methoxyphenyl)-4-(3-methylbutyl)-4,5-dihydro-imidazol-1-yl]-(4-pyrrolidin-1-yl-piperidin-1-yl)methanon; und
[5-(4-Chlorphenyl)-4-(2-cyclohexylethyl)-2-(2-ethoxy-4-methoxyphenyl)-4,5-dihydro-imidazol-1-yl]-(4-pyrrolidin-1-yl-piperidin-1-yl)methanon.

13. Ein Arzneimittel, umfassend mindestens eine Verbindung ausgewählt aus einer Verbindung der Formel I gemäß Anspruch 1 oder ein pharmazeutisch verträgliches Salz oder ein pharmazeutisch verträglicher Ester davon und ein pharmazeutisch verträglicher Exzipient.

14. Verbindungen gemäß einem der Ansprüche 1 bis 13 als Medikament.

15. Verwendung einer Verbindung der Formel I gemäß Anspruch 1 oder ein pharmazeutisch verträgliches Salz oder ein pharmazeutisch verträglicher Ester davon zur Herstellung eines Medikaments zur Behandlung einer Erkrankung, die auf der Wechselwirkung des MDM2-Proteins mit einem p53-artigen Peptid beruht.

16. Verwendung gemäß Anspruch 15, wobei die Erkrankung eine zellproliferative Störung ist.

## Revendications

1. Au moins un composé de formule I 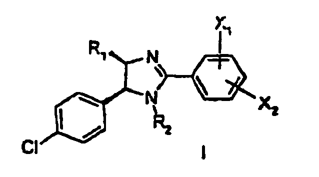 dans laquelle
X₁ et X₂ sont choisis chacun indépendamment dans le groupe constitué par l'hydrogène, -OR₃, -SR₄, -NR₅R₆, -CONR₅R₆, -COOR₇, halogène, nitro, trifluorométhyle, alkyle en C₁-C₆, alkyle en C₁-C₆ substitué par R₈, cycloalkyle en C₃-C₈;
R₁ est choisi dans le groupe constitué par alkyle en C₁-C₈, alkyle en C₁-C₄ lié à un cycloalkyle en C₄-C₈ et cycloalkyle en C₄-C₈;
R₂ est H ou -C=OR₉;
R₃ est choisi dans le groupe constitué par l'hydrogène, alkyle en C₁-C₆, alkyle en C₁-C₆ substitué par R₈, et cycloalkyle en C₃-C₈;
R₄ est choisi dans le groupe constitué par l'hydrogène et alkyle en C₁-C₆;
R₅ et R₆ sont choisis chacun indépendamment dans le groupe constitué par l'hydrogène, alkyle en C₁-C₆ et cycloalkyle en C₃-C₈; ou
R₅ et R₆ forment ensemble, avec les deux atomes de carbone et les liaisons entre eux provenant du cycle benzène auquel ils sont liés, un cycle choisi parmi un cycle insaturé de 5 ou 6 chaînons ou un cycle saturé de 5 ou 6 chaînons contenant au moins un hétéroatome choisi parmi S, N et O;
R₇ est choisi dans le groupe constitué par l'hydrogène, alkyle en C₁-C₆ et cycloalkyle en C₃-C₈;
R₈ est choisi dans le groupe constitué par -CONR₅R₆, -NR₅R₆, COOR₇, aryle, halogène, alcoxy en C₁-C₆, morpholinyle et des hétérocycles;
R₉ est choisi parmi alkyle en C₁-C₄, -CH=CHCOOH, -NHCH₂CH₂R₁₀, -N(CH₂CH₂OH)CH₂CH₂OH, -N(CH₃)CH₂CH₂NCH₃, -N(CH₃)CH₂CH₂N(CH₃)CH₃, des cycles saturés de 4, 5 et 6 chaînons, et des cycles de 5 et 6 chaînons saturés et insaturés contenant au moins un hétéroatome, l'hétéroatome étant choisi parmi S, O et N, et étant éventuellement substitués par un groupe choisi parmi alkyle en C₁-C₆, -C=O-R₁₁, -OH, alkyle en C₁-C₆ substitué par un hydroxy, alkyle en C₁-C₆ substitué par -NH₂, N-alkyle en C₁-C₆, -SO₂CH₃, =O, -CH₂C=OCH₃, et des cycles saturés de 5 et 6 chaînons contenant au moins un hétéroatome choisi parmi S, N et O;
R₁₀ est choisi dans le groupe constitué par -N(CH₃)CH₃, -NHCH₂CH₂NH₂, -NH₂, morpholinyle et pipérazinyle; et
R₁₁ est choisi parmi H, alkyle en C₁-C₆, -NH₂, -N-alkyle en C₁-C₆, alkyle en C₁-C₆ substitué par hydroxy, et alkyle en C₁-C₆ substitué par NH₂,
où chaque cycloalkyle représente un groupe hydrocarboné aliphatique cyclique non aromatique, partiellement ou complètement insaturé;
ou un de ses sels ou esters pharmaceutiquement acceptables.

2. Composé selon la revendication 1, dans lequel R₁ est un alkyle en C₁-C₈.

3. Composé selon la revendication 1, dans lequel R₁ est un alkyle en C₁-C₄ lié à un cycloalkyle en C₄-C₈.

4. Composé selon la revendication 1, dans lequel R₁ est un cycloalkyle en C₄-C₈.

5. Composé selon la revendication 1, dans lequel X₁ est choisi dans le groupe constitué par éthoxy, isopropoxy, 2-fluoroéthoxy et -OCH₂CF₃ en position ortho.

6. Composé selon la revendication 1, dans lequel X₂ est choisi dans le groupe constitué par méthoxy, éthoxy et trifluorométhyle en position para.

7. Composé selon la revendication 1, dans lequel R₉ est choisi dans le groupe constitué par morpholinyle, pipérazinyle, pipéridinyle, cyclopentyle, cyclohexyle, thiophényle, isoxazolyle, furanyle, pipérazinyle substitué par au moins un groupe choisi parmi alkyle en C₁-C₃, alcoxy en C₁-C₂, -C=OCH₃, -SO₂CH₃, -C=O, -OH, -CH₂NH₂, -C=OCH₂NH₂, -C=OCH₂OH, -C=OC(OH)CH₂OH, -CH₂C(OH)-CH₂OH, -C=ON(CH₂-)₂, -C=ONH₂, -C=ON(CH₃)CH₃, -C=OCH(CH₃)₂, -CH₂C=OCH₃, -CH₂CH(OH)CH₃, -CH(CH₃)CH(OH)CH₃ et pipéridinyle substitué par au moins un groupe choisi parmi alkyle en C₁-C₃, alcoxy en C₁-C₂, -C=OCH₃, -SO₂CH₃, -C=O, -OH, -CH₂NH₂, -C=OCH₂NH₂, -C=OCH₂OH, -C=OC(OH)CH₂OH, -CH₂C(OH)-CH₂OH, -C=ON(CH₂)₂, -C=ONH₂, et -C=ON(CH₃)CH₃, -N(CH₃)CH₃, pyrrolidinyle et pipéridinyle.

8. Composé selon la revendication 1, qui est choisi dans le groupe constitué par:
le 5-(4-chlorophényl)-4-cyclopentylméthyl-2-(2-éthoxy-4-méthoxyphényl)-4,5-dihydro-1H-imidazole;
le 5-(4-chlorophényl)-2-(2-éthoxy-4-méthoxyphényl)-4-isobutyl-4,5-dihydro-1H-imidazole;
le 5-(4-chlorophényl)-4-cyclohexyl-2-(2-éthoxy-4-méthoxyphényl)-4,5-dihydro-1H-imidazole;
le 5-(4-chlorophényl)-2-(2-éthoxy-4-méthoxyphényl)-4-pentyl-4,5-dihydro-1H-imidazole;
le 4-butyl-5-(4-chlorophényl)-2-(2-éthoxy-4-méthoxyphényl)-4,5-dihydro-1H-imidazole;
le 5-(4-chlorophényl)-4-cyclohexylméthyl-2-(2-éthoxy-4-méthoxyphényl)-4,5-dihydro-1H-imidazole;
le 5-(4-chlorophényl)-2-(2-éthoxy-4-méthoxyphényl)-4-(3-méthylbutyl)-4,5-dihydro-1H-imidazole;
le 5-(4-chlorophényl)-4-(2-cyclohexyléthyl)-2-(2-éthoxy-4-méthoxyphényl)-4,5-dihydro-1H-imidazole;
le trifluoroacétate du 5-(4-chlorophényl)-2-(2-éthoxy-4-méthoxyphényl)-4-méthyl-4,5-dihydro-1H-imidazole; et
le 5-(4-chlorophényl)-2-(2-éthoxy-4-méthoxyphényl)-4-propyl-4,5-dihydro-1H-imidazole.

9. Composé selon la revendication 1, qui est choisi dans le groupe constitué par:
le trifluoroacétate de 5-(4-chlorophényl)-4-cyclopentyl-2-(2-éthoxy-4-méthoxyphényl)-4,5-dihydro-1H-imidazole;
le 5-(4-chlorophényl)-2-(2-éthoxy-4-méthoxyphényl)-4-éthyl-4,5-dihydro-1H-imidazole;
le 5-(4-chlorophényl)-4-cyclopentylméthyl-2-(2,4-diéthoxyphényl)-4,5-dihydro-1H-imidazole;
le 5-(4-chlorophényl)-2-[2-(2-fluoroéthoxy)-4-méthoxyphényl]-4-propyl-4,5-dihydro-1H-imidazole;
le 5-(4-chlorophényl)-2-(2-isopropoxy-4-méthoxyphényl)-4-propyl-4,5-dihydro-1H-imidazole;
le trifluoroacétate de [5-(4-chlorophényl)-4-cyclopentylinethyl-2-(2-éthoxy-4-méthoxyphényl)-4,5-dihydroimidazol-1-yl]-(4-méthylpipérazin-1-yl)méthanone;
la 4-[5-(4-chlorophényl)-2-(2-éthoxy-4-méthoxyphényl)-4-propyl-4,5-dihydroimidazole-1-carbonyl]pipérazin-2-one;
le trifluoroacétate de [5-(4-chlorophényl)-2-(2-éthoxy-4-méthoxyphényl)-4-propyl-4,5-dihydroimidazol-1-yl]-(4-pyrrolidin-1-ylpipéridin-1-yl)méthanone;
la [5-(4-chlorophényl)-2-(2-éthoxy-4-méthoxyphényl)-4-propyl-4,5-dihydroimidazol-1-yl]-(4-méthylpipérazin-1-yl)méthanone;
le trifluoroacétate de [5-(4-chlorophényl)-4-cyclopentylméthyl-2-(2-éthoxy-4-méthoxyphényl)-4,5-dihydroimidazol-1-yl]-(4-pyrrolidin-1-ylpipéridin-1-yl)méthanone.

10. Composé selon la revendication 1, qui est choisi dans le groupe constitué par:
la 4-[5-(4-chlorophényl)-4-cyclopentylméthyl-2-(2-éthoxy-4-méthoxyphényl)-4,5-dihydroimidazole-1-carbonyl]pipérazin-2-one;
la [5-(4-chlorophényl)-2-(2-éthoxy-4-méthoxyphényl)-4-éthyl-4,5-dihydroimidazol-1-yl]-(4-méthylpipérazin-1-yl)méthanone;
la [5-(4-chlorophényl)-2-(2-éthoxy-4-méthoxyphényl)-4-éthyl-4,5-dihydroimidazol-1-yl]-(4-pyrrolidin-1-ylpipéridin-1-yl)méthanone;
la 4-[5-(4-chlorophényl)-2-(2-éthoxy-4-méthoxyphényl)-4-éthyl-4,5-dihydroimidazole-1-carbonyl]pipérazin-2-one;
la [5-(4-chlorophényl)-4-cyclopentyl-2-(2-éthoxy-4-méthoxyphényl)-4,5-dihydroimidazol-1-yl]-(4-méthylpipérazin-1-yl)méthanone;
la [5-(4-chlorophényl)-4-cyclopentyl-2-(2-éthoxy-4-méthoxyphényl)-4,5-dihydroimidazol-1-yl]-(4-pyrrolidin-1-ylpipéridin-1-yl)méthanone;
la 4-[5-(4-chlorophényl)-4-cyclopentyl-2-(2-éthoxy-4-méthoxyphényl-4,5-dihydroimidazole-1-carbonyl]pipérazin-2-one;
la [5-(4-chlorophényl)-2-(2-éthoxy-4-méthoxyphényl)-4-méthyl-4,5-dihydroimidazol-1-yl]-(4-pyrrolidin-1-ylpipéridin-1-yl)méthanone;
la [5-(4-chlorophényl)-2-(2-éthoxy-4-méthoxyphényl)-4-méthyl-4,5-dihydroimidazol-1-yl]-(4-méthylpipérazin-1-yl)méthanone; et
la 4-[5-(4-chlorophényl)-2-(2-éthoxy-4-méthoxyphényl)-4-méthyl-4,5-dihydroimidazole-1-carbonyl]pipérazin-2-one.

11. Composé selon la revendication 1, qui est choisi dans le groupe constitué par:
la [5-(4-chlorophényl)-2-(2-éthoxy-4-méthoxyphényl)-4-isobutyl-4,5-dihydroimidazol-1-yl]-(4-méthylpipérazin-1-yl)méthanone;
la 4-[5-(4-chlorophényl)-2-(2-éthoxy-4-méthoxyphényl)-4-isobutyl-4,5-dihydroimidazole-1-carbonyl]pipérazin-2-one;
la [5-(4-chlorophényl)-2-(2-éthoxy-4-méthoxyphényl)-4-isobutyl-4,5-dihydroimidazol-1-yl]-(4-pyrrolidin-1-ylpipéridin-1-yl)méthanone;
la [5-(4-chlorophényl)-4-cyclohexyl-2-(2-éthoxy-4-méthoxyphényl)-4,5-dihydroimidazol-1-yl]-(4-méthylpipérazin-1-yl)méthanone;
la 4-[5-(4-chlorophényl)-4-cyclohexyl-2-(2-éthoxy-4-méthoxyphényl-4,5-dihydroimidazole-1-carbonyl]pipérazin-2-one;
la [5-(4-chlorophényl)-4-cyclohexyl-2-(2-éthoxy-4-méthoxyphényl)-4,5-dihydroimidazol-1-yl]-(4-pyrrolidin-1-ylpipéridin-1-yl)méthanone;
le trifluoroacétate de [5-(4-chlorophényl)-2-(2-isopropoxy-4-méthoxyphényl)-4-propyl-4,5-dihyhoimidazol-1-yl]-(4-pyrrolidin-1-ylpipéridin-1-yl)méthanone;
le trifluoroacétate de [5-(4-chlorophényl)-2-(2-isopropoxy-4-méthoxyphényl)-4-propyl-4,5-dihydroimidazol-1-yl]pipérazin-1-ylméthanone;
le trifluoroacétate de [5-(4-chlorophényl)-4-cyclopentylméthyl-2-(2,4-diéthoxyphényl)-4,5-dihydroimidazol-1-yl]-(4-pyrrolidin-1-ylpipéridin-1-yl)méthanone; et
le trifluoroacétate de{5-(4-chlorophényl)-2-[2-(2-fluoroéthoxy)-4-méthoxyphényl]-4-propyl-4,5-dihydro-1H-imidazol-1-yl}-(4-pyrrolidin-1-ylpipéridin-1-yl)méthanone.

12. Composé selon la revendication 1, qui est choisi dans le groupe constitué par:
la 1-{4-[5-(4-chlorophényl)-2-(2-isopropoxy-4-méthoxyphényl)-4-propyl-4,5-dihydroimidazole-1-carbonyl]pipérazin-1-yl} éthanone;
le trifluoroacétate de [5-(4-chlorophényl)-2-(2-isopropoxy-4-méthoxyphényl)-4-propyl-4,5-dihydroimidazol-1-yl]-[4-(2-hydroxyéthyl)pipérazin-1-yl]méthanone;
la 1-{4-[5-(4-chlorophényl)-2-(2-éthoxy-4-méthoxyphényl)-4-isobutyl-4,5-dihydroimidazole-1-carbonyl]pipérazin-1-yl}éthanone;
le trifluoroacétate de [5-(4-chlorophényl)-2-(2-éthoxy-4-méthoxyphényl)-4-isobutyl-4,5-dihydroimidazol-1-yl]-[4-(2-hydroxyéthyl)pipérazin-1-yl]méthanone;
le trifluoroacétate de [5-(4-chlorophényl)-2-(2-éthoxy-4-méthoxyphényl)-4-(3-méthylbutyl)-4,5-dihydroimidazol-1-yl]pipérazin-1-ylméthanone;
la 4-[5-(4-chlorophényl)-2-(2-éthoxy-4-méthoxyphényl)-4-(3-méthylbutyl)-4,5-dihydroimidazole-1-carbonyl]pipérazin-2-one;
la [5-(4-chlorophényl)-2-(2-éthoxy-4-méthoxyphényl)-4-pentyl-4,5-dihydroimidazol-1-yl] -(4-pyrrolidin-1-ylpipéridin-1-yl)méthanone;
la [4-butyl-5-(4-chlorophényl)-2-(2-éthoxy-4-méthoxyphényl)-4,5-dihydroimidazol-1-yl]-(4-pyrrolidin-1-ylpipéridin-1-yl)méthanone;
la [5-(4-chlorophényl)-4-cyclohexylméthyl-2-(2-éthoxy-4-méthoxyphényl)-4,5-dihydroimidazol-1-yl]-(4-pyrrolidin-1-ylpipéridin-1-yl)méthanone;
la [5-(4-chlorophényl)-2-(2-éthoxy-4-méthoxyphényl)-4-(3-méthylbutyl)-4,5- dihydroimidazol-1-yl]-(4-pyrrolidin-1-ylpipéridin-1-yl)méthanone; et
la [5-(4-chlorophényl)-4-(2-cyclohexyléthyl)-2-(2-éthoxy-4-méthoxyphényl)-4,5-dihydroimidazol-1-yl]-(4-pyrrolidin-1-ylpipéridin-1-yl)méthanone.

13. Composition pharmaceutique qui comprend au moins un composé choisi parmi un composé de formule I selon la revendication 1 ou un de ses sels ou esters pharmaceutiquement acceptables, et un excipient pharmaceutiquement acceptable.

14. Composés selon l'une quelconque des revendications 1 à 13 en tant que médicaments.

15. Utilisation d'un composé de formule I selon la revendication 1, ou d'un de ses sels ou esters pharmaceutiquement acceptables, pour la préparation d'un médicament destiné au traitement d'une maladie basée sur l'interaction de la protéine MDM2 avec un peptide de type p53.

16. Utilisation selon la revendication 15, dans laquelle la maladie est un trouble de la prolifération de cellules.
